(19) Europäisches Patentamt
European Patent Office
Office européen des brevets

(11) Publication number: 0 237 967 B1

# (12) EUROPEAN PATENT SPECIFICATION

(45) Date of publication of patent specification: 15.11.95

(51) Int. Cl.⁶: **C07K 14/00**, C12N 15/00, A61K 38/00

(21) Application number: 87103609.1

(22) Date of filing: 12.03.87

Divisional application 93107660.8 filed on 12/03/87.

(54) **IL-1 beta derivatives and drugs.**

(30) Priority: 14.03.86 JP 57885/86
20.06.86 JP 145830/86
09.07.86 JP 161184/86
27.08.86 JP 200324/86

(43) Date of publication of application:
**23.09.87 Bulletin 87/39**

(45) Publication of the grant of the patent:
**15.11.95 Bulletin 95/46**

(84) Designated Contracting States:
**CH DE FR GB IT LI NL SE**

(56) References cited:

**Journal of clinical immunology, vol. 5 no. 5, 1985, pages 287-297, C A Dinarello.**

**Immunobiol., vol. 172, September 1986, pages 301-315, C A Dinarello**

**Biochemical and Biophysical Research Communications, vol. 141, no. 1, November 26, 1986, pages 285-291, K Sato et al.**

(73) Proprietor: **OTSUKA PHARMACEUTICAL CO., LTD.**
**9, Kandatsukasa-cho 2-chome**
**Chiyoda-ku**
**Tokyo 101 (JP)**

(72) Inventor: **Nakai, Satoru**
**67-4, Aza-Minamikawamuko**
**Hiroshima**
**Matsushige-cho**
**Itano-gun**
**Tokushima-ken (JP)**
Inventor: **Kaneta, Mayumi**
**1-81, Higashinakasu**
**Nakamura**
**Kitajima-cho**
**Itano-gun**
**Tokushima-ken (JP)**
Inventor: **Kikumoto, Yoshikazu Haitsu-Datemusume B-302**
**57-1, Aza-Hachigami**
**Sasakino**
**Matsushige-cho**
**Itano-gun**
**Tokushima-ken (JP)**

EP 0 237 967 B1

Lymphokine Research, vol. 2. no. 3, 1983, pages 97-102, Mary Ann Liebert Inc., Publ. R F Kampschmidt

Inventor: **Hong, Yeong-Man**
**Sanraunjihausu 105**
**129-4, Miyanonishi**
**Kitahama**
**Muya-cho**
**Naruto-shi**
**Tokushima-ken (JP)**
Inventor: **Kawai, Kazuyoshi Haitsu Soreiyu**
**503**
**130-2, Aza-Mitsuhokaitaku**
**Mitsuho**
**Matsushige-cho**
**Itano-gun**
**Tokushima-ken (JP)**
Inventor: **Takegata, Setsuko**
**1090-18, Kagasuno**
**Kawauchi-cho**
**Tokushima-shi**
**Tokushima-ken (JP)**
Inventor: **Ishii, Kiyoshi**
**39-46, Aza-Sakafuji**
**Sumiyoshi**
**Aizumi-cho**
**Itano-gun**
**Tokushima-ken (JP)**
Inventor: **Yanagihara, Yasuo**
**891-6, Oomatsu**
**Kawauchi-cho**
**Tokushima-shi**
**Tokushima-ken (JP)**
Inventor: **Hirai, Yoshikatsu**
**5-49, Aza-Ekogawa**
**Shinkirai**
**Kitajima-cho**
**Itano-gun**
**Tokushima-ken (JP)**

(74) Representative: **VOSSIUS & PARTNER**
**Postfach 86 07 67**
**D-81634 München (DE)**

## Description

The present invention relates to novel polypeptides, and more particularly to novel derivatives of interleukin-1$\beta$ (hereinafter referred to as IL-1$\beta$) and to the medicinal use of said novel derivatives.

The Second International Lymphokine Workshop decided to adopt a unified name, interleukin-1 (IL-1), for the physiologically active substances which had been referred to as lymphocyte activating factor (LAF), mitogenic protein, helper peak-1, T-cell replacing factor III (TRF-III), T-cell replacing factor M$\phi$ (TRFM), B-cell activating factor, B-cell differation factor, etc. (Cellular Immunol., 48, 433-436 (1979)). This decision is based on the reason that these physiologically active substances can not be distinguished from one another as different substances but are expressed variously merely with reference to physiological activities as interpreted from different angles.

Further it is reported that IL-1 activates T lymphocytes and B lymphocytes, has activity to promote production of interleukin-2 and antibodies, acts on liver tissues to promote protein synthesis and possesses activity to promote production of prostaglandins (see Reviews of Infectious Disease, Vol 6, No.1, 51-59 (1984), New England J. of Med., 311, 1413 (1984), etc).

Whereas IL-1 itself still remains to be clarified as a substance, it is only recently that reports are made on the presence of two different genes coding for polypeptides having LAF activity or precursor thereof (Proc. Natl. Acad. Sci., Vol. 81, 7907-7911 (1984), Nature, Vol. 315, 641 (1985), Nucleic Acid Research, Vol. 13 (16), 5869 (1985)). These reports mention "IL-1$\alpha$" having a sequence of 159 amino acids and "IL-1$\beta$" comprising 153 amino acids which are postulated from the base sequences of the two genes.

Nevertheless, research has yet to be made to clarify the relation between said activities assayed using a conditioned medium or so called partially purified IL-1 and the polypeptide postulated from the above base sequences.

Known IL-1 as a physiologically active substance is also thought to be identical with an endogeneous pyrogen (EP) and is known to exhibit a pyrogenic property (see Nature, 304, 449 (1983); Cell Immunol., 63, 164 (1981); J. Exp. Med., 150, 709 (1979); J. Immunol., 130, (6) 2708 (1983); same, 132 (3) 1311 (1984); etc.). This indicates that IL-1, if available as a uniform substance, to say nothing of physiologically active conventional IL-1, is difficult to use for medical applications.

An object of the present invention is to provide polypeptides which are novel and useful IL-1$\beta$ derivatives effectively usable for medicinal applications.

Another object of the invention is to provide a medicinal use for polypeptides which are novel IL-1$\beta$ derivatives.

Another object of the invention is to provide a gene coding for a novel IL-1$\beta$ derivative and a process for preparing the IL-1$\beta$ derivative,i.e., a polypeptide, by gene engineering techniques using the gene.

The above and other objects of the invention will become apparent from the following description.

The IL-1$\beta$ derivative (polypeptide) of the invention is characterized in that the derivative has a modified amino acid sequence fulfilling at least one of the following requirements a) to c) together with requirement d) in the amino acid sequence of interleukin-1$\beta$ represented by the formula (A).

Formula (A)

```
                         15                                    20
Arg - Asp - Ser - Gln - Gln - Lys - Ser - Leu - Val - Met-

                         25                                    30
Ser - Gly - Pro - Tyr - Glu - Leu - Lys - Ala - Leu - His-

                         35                                    40
Leu - Gln - Gly - Gln - Asp - Met - Glu - Gln - Gln - Val-

                         45                                    50
Val - Phe - Ser - Met - Ser - Phe - Val - Gln - Gly - Glu-

                         55                                    60
Glu - Ser - Asn - Asp - Lys - Ile - Pro - Val - Ala - Leu-

                         65                                    70
Gly - Leu - Lys - Glu - Lys - Asn - Leu - Tyr - Leu - Ser-

                         75                                    80
Cys - Val - Leu - Lys - Asp - Asp - Lys - Pro - Thr - Leu-

                         85                                    90
Gln - Leu - Glu - Ser - Val - Asp - Pro - Lys - Asn - Tyr-

                         95                                    100
Pro - Lys - Lys - Lys - Met - Glu - Lys - Arg - Phe - Val-

                         105                                   110
Phe - Asn - Lys - Ile - Glu - Ile - Asn - Asn - Lys - Leu-

                         115                                   120
Glu - Phe - Glu - Ser - Ala - Gln - Phe - Pro - Asn - Trp-

                         125                                   130
Tyr - Ile - Ser - Thr - Ser - Gln - Ala - Glu - Asn - Met-

                         135                                   140
Pro - Val - Phe - Leu - Gly - Gly - Thr - Lys - Gly - Gly-

                         145                                   150
Gln - Asp - Ile - Thr - Asp - Phe - Thr - Met - Gln - Phe-


Val - Ser - Ser
```

a) At least one amino acid residue selected from among Ala at the 1-position, Val at the 3-position, Arg at the 4-position, Ser at the 5-position, Cys at the 8-position, Arg at the 11-position, His at the 30-position, Cys at the 71-position, Lys at the 93-position, Lys at the 97-position, Arg at the 98-position, Phe at the 99-position, Lys at the 103-position, Trp at the 120-position, Tyr at the 121-position and Ser at the 153-position is missing or replaced by another amino acid.

b) The amino acid sequence of Ala at the 1-position to Thr at the 9-position or at least one amino acid residue in this sequence is missing (except that at least one amino acid residue selected from the group consisting of Ala at the 1-position, Val at the 3-position, Arg at the 4-position, Ser at the 5-position and Cys at the 8-position is missing as stated in the requirement a)).

c) The amino acid sequence of Lys at the 103 position to Ser at the 153-position or at least one amino acid residue in this sequence is missing (except that at least one amino acid residue selected from the group consisting of Lys at the 103-position, Trp at the 120-position, Tyr at the 121-position and Ser at the 153-position is missing as stated in the requirement a)).

d) Met, or the amino acid sequence of Met at the 1'-position to Asp at the 116'-position represented by the following formula (B), or a portion of the sequence of the formula (B) toward its C terminal is attached to the N terminal of the formula (A).

Formula (B)

```
                              5'                                    10'
Met - Ala - Glu - Val - Pro - Glu - Leu - Ala - Ser - Glu-

                              15'                                   20'
Met - Met - Ala - Tyr - Tyr - Ser - Gly - Asn - Glu - Asp-

                              25'                                   30'
Asp - Leu - Phe - Phe - Glu - Ala - Asp - Gly - Pro - Lys-

                              35'                                   40'
Gln - Met - Lys - Cys - Ser - Phe - Gln - Asp - Leu - Asp-

                              45'                                   50'
Leu - Cys - Pro - Leu - Asp - Gly - Gly - Ile - Gln - Leu-

                              55'                                   60'
Arg - Ile - Ser - Asp - His - His - Tyr - Ser - Lys - Gly-

                              65'                                   70'
Phe - Arg - Gln - Ala - Ala - Ser - Val - Val - Val - Ala-

                              75'                                   80'
Met - Asp - Lys - Leu - Arg - Lys - Met - Leu - Val - Pro-

                              85'                                   90'
Cys - Pro - Gln - Thr - Phe - Gln - Glu - Asn - Asp - Leu-

                              95'                                   100'
Ser - Thr - Phe - Phe - Pro - Phe - Ile - Phe - Glu - Glu-

                              105'                                  110'
Glu - Pro - Ile - Phe - Phe - Asp - Thr - Trp - Asp - Asn-

                              115'
Glu - Ala - Tyr - Val - His - Asp
```

provided that Cys at position 71 only is not replaced by Ser.

Amino acids and peptides are herein referred to by symbols according to the nomenclature or rules recommended by IUPAC and IUAC-IUB or by symbols conventionally used in the art. The nucleic acids in base sequences are also similarly expressed. The amino acid number or position as used herein with reference to the polypeptide of the present invention is based on the amino acid sequence of IL-1β, i.e., the amino acid sequence of the formula (A), unless otherwise stated, even in the case where there is a deficiency or extension. However, the primed number or position is based on the amino acid sequence of the formula (B).

The IL-1β derivative of the present invention is a novel polypeptide having an amino acid sequence which fulfills at least one, of the foregoing requirements a) to c) in the amino acid sequence of IL-1β represented by the formula (A).

Preferred examples of IL-1β derivatives, i.e. polypeptides, of the invention are as follows.

1) A polypeptide having the amino acid sequence of the formula (A) wherein at least Ala at the 1-position is missing or replaced by another amino acid.

2) A polypeptide having the amino acid sequence of the formula (A) wherein at least Val at the 3-position is missing or replaced by another amino acid.

3) A polypeptide having the amino acid sequence of the formula (A) wherein at least Arg at the 4-position is missing or replaced by another amino acid.

4) A polypeptide having the amino acid sequence of the formula (A) wherein at least Ser at the 5-position is missing or replaced by another amino acid.

5) A polypeptide having the amino acid sequence of the formula (A) wherein at least Cys at the 8-position is missing or replaced by another amino acid.

6) A polypeptide having the amino acid sequence of the formula (A) wherein at least Arg at the 11-position is missing or replaced by another amino acid.

7) A polypeptide having the amino acid sequence of the formula (A) wherein at least His at the 30-position is missing or replaced by another amino acid.

8) A polypeptide having the amino acid sequence of the formula (A) wherein at least Cys at the 71-position is missing or replaced by another amino acid.

9) A polypeptide having the amino acid sequence of the formula (A) wherein at least Lys at the 93-position is missing or replaced by another amino acid.

10) A polypeptide having the amino acid sequence of the formula (A) wherein at least Lys at the 97-position is missing or replaced by another amino acid.

11) A polypeptide having the amino acid sequence of the formula (A) wherein at least Arg at the 98-position is missing or replaced by another amino acid.

12) A polypeptide having the amino acid sequence of the formula (A) wherein at least Phe at the 99-position is missing or replaced by another amino acid.

13) A polypeptide having the amino acid sequence of the formula (A) wherein at least Lys at the 103-position is missing or replaced by another amino acid.

14) A polypeptide having the amino acid sequence of the formula (A) wherein at least Trp at the 120-position is missing or replaced by another amino acid.

15) A polypeptide having the amino acid sequence of the formula (A) wherein at least Tyr at the 121-position is missing or replaced by another amino acid.

16) A polypeptide having the amino acid sequence of the formula (A) wherein at least Ser at the 153-position is missing or replaced by another amino acid.

17) A polypeptide having the amino acid sequence of the formula (A) which is missing at least in the amino acid sequence of Ala at the 1-position to Val at the 3-position, the amino acid sequence of Ala at the 1-position to Leu at the 6-position or the amino acid sequence of Ala at the 1-position to Thr at the 9-position.

18) A polypeptide having the amino acid sequence of the formula (A) which is missing at least in the amino acid sequence of Val at the 151-position to Ser at the 153-position, the amino acid sequence of Gln at the 149-position to Ser at the 153-position, the amino acid sequence of Asp at the 145-position to Ser at the 153-position, the amino acid sequence of Gln at the 141-position to Ser at the 153-position, the amino acid sequence of Tyr at the 121-position to Ser at the 153-position, or the amino acid sequence of Lys at the 103-position to Ser at the 153-position.

19) A polypeptide having the amino acid sequence of the formula (A) the N terminal of which has attached thereto at least Met, the amino acid sequence of Met at the 77′-position to Asp at the 116′-position, the amino acid sequence of Met at the 71′-position to Asp at the 116′-position, the amino acid sequence of Met at the 32′-position to Asp at the 116′-position or the amino acid sequence of Met at the 1′-position to Asp at the 116′-position of the Formula (B).

While the IL-1$\beta$ derivatives of the present invention include polypeptides having the amino acid sequence of IL-1$\beta$ wherein the specific amino acid at the specific position is replaced by some other amino acid, the replacing amino acid may be any of $\alpha$-amino acids constituting human proteins. However, Cys is likely to form a disulfide linkage intra- or inter-molecularly with its SH group. In view of this, the desirable amino acids are those other than Cys. Examples of more desirable amino acids are Gly for replacing the 4-position Arg; Ser or Ala for the 8-position Cys; Gln for the 11-position Arg; Tyr for the 30-position His; Ser, Ala or Val for the 71-position Cys; Leu for the 93-position Lys; Leu for the 98-position Arg; Gln for the 103-position Lys; Arg for the 120-position Trp; and Gln for the 121-position Tyr.

The IL-1 derivatives of the invention obtained in accordance with the invention have, for example, physiological activities such as LAF activity, activity to inhibit growth of tumor cells (GIF activity), i.e. activity to specifically inhibit growth of tumor cells, activity to promote production of various cytokines such as

6

colony stimulating factor (CSF), interferon (IFN), interleukin-2 (IL-2) and interleukin-3 (IL-3), i.e. activity for example on human cells to greatly promote production of such cytokines, anti-inflammatory activity, activity for example to effectively inhibit progress of arthritis when administered to model animals with arthritis, and activity to prevent radiation injury, i.e. activity to prevent the possible living body disorders or serious side effects that would result from systemic radiation irradiation during bone marrow transplant, radiation irradiation for treatment of cancers and radiation accident. Accordingly, the IL-1$\beta$ derivatives of the invention are very useful as immune system stimulants, for example, for promoting production of antibodies and enhancing the effect of vaccines, antitumor agents, agents for promoting production of cytokines such as CSF, IFN, IL-2 and IL-3, anti-inflammatory agents, agents for preventing radiation sickness and other like medicinal agents.

The derivatives of the present invention are excellent in at least one of the activities specified above and/or in being low in toxicity and in side effect.

The derivatives of the invention are effective especially as CSF production promoting agents. When administered to man, the derivative effectively cures granulocytopenia due to impaired formation of bone marrow resulting from chemotherapy or radiation therapy for cancers without entailing the likelihood of virus infections or antigen-antibody reaction (granulocytopenia curing drug). The CSF production promoting agent is usable also for preventing and curing various diseases owing to the activity of CSF the production of which is promoted by the agent as contemplated. For example, CSF acts to promote the function of granulocytes and macrophages (Lopez, A. F. et al., J. Immunol., 131, 2983 (1983); Handam, E. et al., same, 122, 1134 (1979) and Vadas, M. A. et al., same, 130, 795 (1983)), so that clinical application is expected of CSF for preventing and curing various infections. Similarly, the CSF production promoting agent is expected to be useful for clinical application.

In recent years, it is noted that compromised hosts with impaired biophylactic ability suffer from so-called opportunistic infections or terminal infections which occur when harmless pathogens become pathogenic. Clinically, these infections are attributable to pathogens including gram-negative bacilli such as Pseudomonas and Serratia, viruses such as Herpes simplex virus (HSV), Varicella-zoster virus (VZV) and Cytomegalovirus (CMV), fungi such as candida albicans, Aspergillus fumigatus and nocardia asteroidea, protozoa such as Pneumocystis carinii and Toxoplasma gondii, etc. Since the antibiotics presently used are not fully effective on the opportunistic infections, it is desired to conduct research on and develop new drugs for such infections. The present IL-1$\beta$ derivatives are effective also for preventing and curing opportunistic infections which occur frequently especially when anticancer drugs are given. For example, they are useful for preventing and curing various infections which develop during chemotherapy of acute leukemia and bone marrow transplant, such as candidosis, cryptococcosis, aspergillosis, zygomycosis, chromomycosis, virus infections, Cytomegalovirus pneumonia and complications of these infections.

Besides the above-mentioned medicinal uses, the IL-1$\beta$ derivatives of the present invention are very useful, for example, for preparing various useful cytokines in vitro from a cell line, etc. because of their activity to promote production of cytokines. Attention has been directed to the preparation in natural-type cytokines, especially those which are glyco-proteins. The present derivatives make it possible to obtain useful cytokines efficiently in large quantities when used as a cytokine production inducing agent.

Among the derivatives of the present invention, those exhibiting high activity are polypeptides of the formula (A) wherein at least Cys at the 71-position is missing or replaced by some other amino acid, especially by an amino acid such as Ser, Ala, Val or the like.

Further the present derivatives of the formula (A) wherein at least Arg at the 4-position or Cys at the 8-position is missing or replaced, and those which are at least deficient in at least one of the amino acids at the 103-position and the following positions have the feature of being low in activity to promote production of prostaglandin E (PGE) and therefore being diminished in side effects such as pyrogenic activity and being low in toxicity, and those wherein at least Arg at the 4-position is missing or replaced have the feature of being higher in activity to promote production of CSF and in anti-inflammatory activity than in GIF activity and LAF activity.

Further the present derivatives having at least the above-specified amino acid(s) or polypeptide attached to the N terminal of the formula (A) also have the feature of being higher in activity to promote production of CSF and antiinflammatory activity than in GIF activity and LAF activity, and are useful as medicinals, especially as oral drugs or suppositories, since they are low in toxicity and exhibit sustained activity.

Moreover, the present derivatives, particularly those wherein Cys at the 8-position and Cys at the 71-position are missing or replaced are excellent in binding activity against the IL-1 receptor under various conditions. More preferable are those wherein the Cys at the 8- and 71-positions is replaced by Ser, Ala and/or Val.

EP 0 237 967 B1

Among the present derivatives wherein the molecule has a lower Cys content than in IL-1$\beta$ or is free from Cys are more preferably in view of the possible unnecessary formation of an intra- or inter-molecular linkage due to the SH group of Cys.

The polypeptides of the present invention can be prepared, for example, by gene engineering techniques using a gene coding for the specific polypeptide of the invention, i.e., by incorporating the gene into a microorganism vector to effect replication, transcription and translation within the cell of the microorganism. This process is advantageous in that it is amenable to mass production.

Although the gene to be used in this process can to totally synthesized through chemical synthesis of nucleic acids by a usual method, for example, by the phosphite triester method (Nature, 310, 105 (1984)) or the like, it is convenient to utilize the gene coding for IL-1$\beta$ or a precursor thereof. By a conventional method involving the above chemical synthesis, the gene is modified to a sequence of nucleic acids coding for the foregoing specific amino acid sequence, whereby the desired gene can be prepared easily.

The gene coding for IL-1$\beta$ or a precursor thereof is already known. We obtained the gene coding for IL-1$\beta$ as disclosed in our copending application No.85116344.4 and succeeded in preparing IL-1$\beta$ by gene engineering techniques using this gene. The series of gene engineering techniques employed will be described in the reference examples to follow.

The above-mentioned modified sequence of nucleic acids (bases) is prepared also by a known procedure, which executed according to the amino acid sequence of the desired polypeptide (Molecular Cloning Cold Spring Harbor Laboratory (1982)).

For example, cleavage, ligation, phosphorylation, etc. of DNA can be carried out by usual methods including treatment with enzymes such as restriction enzymes, DNA ligase, polynucleotidekinase and DNA polymerase, which are readily available as commercial products. The isolation and purification of the gene and nucleic acids included in these methods are conducted also in the usual manner, for example, by agarose gel electrophoresis. As will be described partially later, the gene obtained is replicated using a usual vector. The DNA fragment coding for the desired amino acid sequence and synthetic linkers can be prepared also easily by the above-mentioned chemical synthesis. The codon corresponding to the desired amino acid and to be used in the above methods is known and is selected as desired. A usual method may be used for this purpose, for example, in view of the frequency of use of the codon of the host to be used (Nucl. Acids Res., 9, 43-73 (1981)). Further for the modification of the codon in the nucleic acid sequence concerned, for example, site-specific mutagenesis (Proc. Natl. Acad.. Sci., 81, 5662-5666 (1984)) can be resorted to as usually done which employs a primer comprising a synthetic oligonucleotide coding for the desired modified sequence about 15-30 mer.

The desired gene obtained by the foregoing process can be checked for its base sequence, for example, by the Maxam-Gilbert chemical modification method (Meth. Enzym., 65, 499-560 (1980)) or by the dideoxynucleotide chain termination method using MI3 Phage (Messing, J. and Vieira, J., Gene, 19, 269-276 (1982)).

While the above process and procedures therefor will be described in the reference examples and examples to follow, the process is not specifically limited; any process already known in the art may be used.

Thus, the present invention also provides a novel gene coding for a polypeptide having the above-specified amino acid sequence. (The gene will hereinafter be referred to as the "present gene.")

The polypeptide of the present invention can be prepared by usual known gene recombination techniques using the present gene. More specifically, it is produced by preparing a recombinant DNA which can express the present gene in host cells, transforming the DNA into the host cell and incubating the transformant.

Useful host cells can be either eucaryotic or procaryotic cells. The eucaryotic cells include cells of vertebrate animals, yeasts, etc. Generally used as cells of vertebrate animals are, for example, COS cells which are cells of monkey (Y. Gluzman, Cell, 23, 175-182 (1981)), dihydrofolate reductase defective strain of Chinese hamster ovary cell (G. Urlaub and L.A., Chasin, Proc. Natl. Acad. Sci., U.S.A., 77, 4216-4220 (1980)), etc., while useful cells are not limited to these cells. Useful expression vectors of vertebrate cells are those having a promotor positioned upstream of the gene to be expressed, RNA splicing sites, polyadenylation site, transcription termination sequence, etc. These vectors may further have a replication origin when required. Examples of useful expression vectors include pSV2dhfr having an initial promotor of SV40 (S. Subramani, R. Mulligan and P. Berg, Mol. Cell. Biol., 1(9), 854-864), which is not limitative.

Yeasts are widely used as eucaryotic microorganisms, among which those of the genus Saccharomyces are generally usable. Examples of popular expression vectors of yeasts and like eucaryotic microorganisms include pAM82 having a promotor for acid phosphatase gene (A. Miyanohara et al., Proc. Natl. Acad. Sci., U.S.A., 80, 1-5 (1983), etc.

8

E. coli and Bacillus subtilis are generally used as procaryotic hosts. The present invention employs, for example plasmid vectors capable of replication in the host. To express the gene in the vector, expression plasmids can be used which have a promotor and SD (Shine-Dalgarno) base sequence at the upstream of the gene and ATG required for initiating protein synthesis. Widely used as host E. coli is E. coli Kl2 strain. pBR322 is a vector which is generally used. However, these are not limitative, and various known strains and vectors are usable. Examples of promotors usable are tryptophan promotor, $P_L$ promotor, lac promotor, lpp promotor, etc. The gene can be expressed with use of any of these promotors.

To describe the procedure with reference to the case wherein tryptophan promotor is used, vector pTMI (Funio Imamoto, Taishya, Vol. 22, 289 (1985)) having tryptophan promotor and SD sequence is used as an expression vector. A gene coding for a desired polypeptide of this invention and having ATG when required is linked to the site of restriction enzyme ClaI which is present downstream from the SD sequence.

Incidentally, not only the direct expression system but a fusion protein expression system is also usable which employs, for example, $\beta$-galactosidase, $\beta$-lactamase or the like.

The expression vector thus obtained is introduced into host cells and thereby transformed by usual methods. For example, cells chiefly in the logarithmic growth phase are collected, treated with $CaCl_2$ and thereby made to readily accept DNA, whereupon the vector is introduced into the cell. With this method, $MgCl_2$ or RbCl can be made present conjointly with the vector so as to achieve an improved transformation efficiency, as is generally known. The cell can be converted to spheroplast or protoplast before transformation.

The desired transformant thus obtained can be incubated in the usual manner, whereby the desired polypeptide is produced and accumulated. The medium for the incubation may be any of those generally used for incubating cells, such as L medium, E medium, M9 medium, etc. Various carbon sources, nitrogen sources, inorganic salts, vitamins, etc. which are usually known can be admixed with these media. When the tryptophan promotor is used, M9 minimum medium, for example, is usable which has admixed therewith Casamino acid for effecting the action of the promotor. A chemical, such as indoleacrylic acid, for enhancing the action of tryptophan promotor can be added to the medium at a suitable stage of incubation.

The desired polypeptide of the present invention can be isolated from the resulting culture and purified by usual methods. It is desirable to extract the polypeptide from the host under a mild condition as by osmotic shock in order to maintain the higher-order structure thereof.

The above isolation or purification method is conducted substantially by the same method as usually used for separating a protein-like substance from such a biological substance. For example, various procedures are usable utilizing the physical or chemical properties of the desired polypeptide. (See for example, "Biological Data Book II," pp. 1175-1259, 1st edition, 1st print, June 23, 1980, published by Kabushiki Kaisha Tokyo Kagakudojin.) Examples of useful procedures are treatment with use of a usual protein precipitating agent, ultra-filtration, molecular sieve chromatography (gel filtration), liquid chromatography, centrifugation, electrophoresis, affinity chromatography, dialysis, and combinations of such procedures.

The desired polypeptide is separated from the supernatant as partially purified. This partial purification is carried out, for example, by a treatment using as a protein precipitating agent an organic solvent such as acetone, methanol, ethanol, propanol or dimethylformamide (DMF), or an acidic reagent such as acetic acid, perchloric acid (PCA) or trichloroacetic acid (TCA), a treatment using a salting-out agent such as ammonium sulfate, sodium sulfate or sodium phosphate and/or ultrafiltration using a dialysis membrane, flat membrane, hollow fiber membrane or the like. These treatments are conducuted in the same manner as usually done under usual conditions.

The roughly purified product thus obtained in then subjected to gel filtration, whereby a fraction exhibiting the activity of the desired substance is collected. Useful gel filtration agents are not limited specifically. Such agents include those made of dextran gel, polyacrylamide gel, agarose gel, polyacrylamide-agarose gel, cellulose or the like. Examples of useful agents commercially available are Sephadex G type, Sephadex LH type, Sepharose type, Sephacryl type (all products of Pharmacia), Cellofine (Chisso Corporation), Biogel P type, Biogel A type (both product of Bio-Rad Lab), Ultro gel-C (LKB), TSK-G type (product of Toyo Soda Mfg. Co., Ltd.), etc.

The polypeptide of the present invention can be isolated from the fraction as a homogeneous substance, for example, by subjecting the fraction to affinity chromatography with use of a hydroxyapatite column, ion exchange column chromatography as of the DEAE, CM or SP method, chromatofocusing method, reverse-phase high-performance liquid chromatography or the like, or to a combination of such methods.

The chromatofocusing method can be carried out by various known procedures. Usable as the column is, for example, PBE94 (Pharmacia Fine Chemicals) or the like, as the starting buffer, for example, imidazole-hydrochloric acid or the like, and the eluent, for example, the mixture of Polybuffer 74 (Pharmacia Fine Chemicals) and hydrochloric acid (pH 4.0) or the like.

The reverse-phase high-performance liquid chromatography can be conducted, for example, with $C_4$ Hi-Pore reverse-phase HPLC column (Bio-Rad Laboratories) or the like, using acetonitrile, trifluoroacetic acid (TFA), water of the like, or a mixture of such solvents as the eluent.

In this way, the IL-$1\beta$ derivative (polypeptide) of the present invention can be obtained upon isolation. The gene coding for IL-$1\beta$ affords IL-$1\beta$ through a similar gene recombination procedure.

The polypeptide of the invention, which has outstanding pharmacological activities as already stated, can be formulated into useful preparations for the aforementioned medicinal uses. Examples of such medicinal preparations include immunostimulators for producing antibodies, enhancing the effect of vaccines and curing immunodeficiency, antitumor agents, cytokine production promotors, anti-inflammatory agents, agents for preventing or curing radiation injury, agents for preventing or curing opportunistic infections, etc. These medicinal preparations are formulated usually in the form of pharmaceutical compositions comprising a pharmacologically effective amount of the present peptide or IL-$1\beta$ and a suitable carrier. Examples of useful pharmaceutical carriers include excipients and diluents such as filler, extender, binder, wetting agent, disintegrator, surfactant, etc. which are generally used for preparing pharmaceuticals of the desired form to be used. The form of the pharmaceutical compositions is not specifically limited insofar as they effectively contain the present polypeptide or IL-$1\beta$ but can be, for example, in the form of tablets, powder, granules, pellets or like solid preparation. Usually, however, it is suitable that the composition be in the form of a solution, suspension, emulsion or the like for injection. Alternatively, such a composition can be a dry product which can be made liquid with addition of a suitable carrier before use. The pharmaceutical compositions mentioned above can be prepared by usual methods.

In accordance with the form of the pharmaceutical composition obtained, the composition is administered via a suitable route. For example, those for injection are given intravenously, intramuscularly, subcutaneously, intracuteneously, intraperitoneally, or otherwise. The solid composition is given orally or intraintestinally. The amount of the active component of the composition and the dosage of the composition are suitably determined according to the method and form of administration, purpose of use, symptoms of the patient, etc. and are not definitely determinable. It is generally desirable to incorporate about 1 to about 80 wt. % of the active component into the pharmaceutical preparation and to give the preparation at a daily dose of about 0.1 $\mu$g to about 10 mg, calculated as the active component, for adults. The preparation need not always be given only once a day but can be given in three to four divided doses daily.

The present invention will be described in greater detail with reference to the following reference examples wherein IL-$1\beta$ was prepared and examples wherein derivatives of the invention were prepared.

In these examples, the following symbols are used for the derivatives of the invention for a simplified description.

| Symbol (polypeptide No.) | IL-1β and derivative |
|---|---|
| I | IL-1β |
| II | [Gly⁴]IL-1β (IL-1β wherein Arg at 4-position is replaced by Gly) |
| III | [Gln¹¹]IL-1β (IL-1β wherein Arg at 11-position is replaced by Gln) |
| IV | [Ser⁸]IL-1β (IL-1β wherein Cys at 8-position is replaced by Ser) |
| V | [Ser⁸, Ser⁷¹]IL-1β (IL-1β wherein Cys at 8-position is replaced by Ser, and Cys at 71-position by Ser) |
| VII | [Gln¹⁰³]IL-1β (IL-1β wherein Lys at 103-position is replaced by Gln) |
| VIII | IL-1β(1-140)-polypeptide (IL-1β wherein the amino acids at 141-position et seq. are deficient) |
| IX | [Gln¹²¹]IL-1β (IL-1β wherein Tyr at 121-position is replaced by Gln) |
| XI | des-Ala¹-IL-1β (IL-1β wherein Ala at 1-position is deficient) |
| XII | [Leu⁹⁸]IL-1β (IL-1β wherein Arg at 98-position is replaced by Leu) |
| XIII | [Gly⁴, Gln¹¹, Leu⁹⁸]IL-1β (IL-1β wherein Arg at 4-position is replaced by Gly, Arg at 11-position by Gln, and Arg at 98-position by Leu) |

XIV     $[\text{Gly}^4, \text{Gln}^{11}]$IL-1β (IL-1β wherein Arg at 4-position is replaced by Gly, and Arg at 11-position by Gln)

XV     $[\text{Gly}^4, \text{Leu}^{98}]$IL-1β (IL-1β wherein Arg at 4-position is replaced by Gly, and Arg at 98-position by Leu)

XVI     $[\text{Gln}^{11}, \text{Leu}^{98}]$IL-1β (IL-1β wherein Arg at 11-position is replaced by Gln, and Arg at 98-position by Leu)

XVII     des-$\text{Arg}^4$-IL-1β (IL-1β wherein Arg at 4-position is deficient)

XVIII     des-$\text{Arg}^{11}$-IL-1β (IL-1β wherein Arg at 11-position is deficient)

XIX     des-$\text{Arg}^{98}$IL-1β (IL-1β wherein Arg at 98-position is deficient)

XX     $[\text{Ala}^8]$IL-1β (IL-1β wherein Cys at 8-position is replaced by Ala)

XXI     des-$\text{Cys}^8$-IL-1β (IL-1β wherein Cys at 8-position is deficient)

XXII     $[\text{Ala}^{71}]$IL-1β (IL-1β wherein Cys at 71-position is replaced by Ala)

XXIII     $[\text{Val}^{71}]$IL-1β (IL-1β wherein Cys at 71-position is replaced by Val)

XXIV     des-$\text{Cys}^{71}$-IL-1β (IL-1β wherein Cys at 71-position is deficient)

XXV     $[\text{Leu}^{93}]$IL-1β (IL-1β wherein Lys at 93-position is replaced by Leu)

XXVI     IL-1β-(1-144)-polypeptide (IL-1β wherein the amino acids at 145-position et seq. are deficient)

XXVII     IL-1β-(1-148)-polypeptide (IL-1β wherein the amino acids at 149-position et seq. are deficient)

XXVIII     $[\text{Arg}^{120}]$IL-1β (IL-1β wherein Trp at 120-position is replaced by Arg)

XXIX          [Tyr$^{30}$]IL-1β (IL-1β wherein His at 30-
              position is replaced by Tyr)

XXX           IL-1β-(1-102)-polypeptide (IL-1β wherein the
              amino acids at 103-position et seq. are
              deficient)

XXXI          (77'-116')-IL-1β (IL-1β having attached to
              its N terminal 77'-116' position amino acid
              sequence of the formula (B))

XXXII         (71'-116')-IL-1β (IL-1β having attached to
              its N terminal 71'-116' position amino acid
              sequence of the formula (B))

XXXIII        (32'-116')-IL-1β (IL-1β having attached to
              its N terminal 32'-116' position amino acid
              sequence of the formula (B))

XXXIV         (1'-116')-IL-1β (IL-1β having attached to its
              N terminal 1'-116' position amino acid
              sequence of the formula (B))

XXXV          IL-1β-(1-120)-polypeptide (IL-1β wherein the
              amino acids at 121-position et seq. are
              missing)

XXXVI         [Ala$^{8}$, Ser$^{71}$]IL-1β (IL-1β wherein Cys at 8-
              position is replaced by Ala, and Cys at 71-
              position by Ser)

XXXVII        [Ala$^{8}$, Ala$^{71}$]IL-1β (IL-1β wherein Cys at 8-
              position is replaced by Ala, and Cys at 71-
              position by Ala)

XXXVIII       [Ala$^{8}$, Val$^{71}$]IL-1β (IL-1β wherein Cys at 8-
              position is replaced by Ala, and Cys at 71-
              position by Val)

XXXIX         IL-1β-(4-153)-polypeptide (IL-1β which is
              deficient in the amino acid sequence of Ala
              at 1-position to Val at 3-position)

XXXX          IL-1β-(7-153)-polypeptide (IL-1β which is
              deficient in the amino acid sequence of Ala
              at 1-position to Leu at 6-position)

XXXXI         IL-1β-(10-153)-polypeptide (IL-1β which is
              deficient in the amino acid sequence of Ala
              at 1-position to Thr at 9-position)

XXXXII    des-Val$^3$-IL-1β (IL-1β wherein Val at 3-position is missing)

XXXXIII   des-Ser$^5$-IL-1β (IL-1β wherein Ser at 5-position is missing)

XXXXIV    des-Lys$^{97}$-IL-1β (IL-1β wherein Lys at 97-position is missing)

XXXXV     des-Phe$^{99}$-IL-1β (IL-1β wherein Phe at 99-position is missing)

XXXXVI    IL-1β-(1-150)-polypeptide (IL-1β wherein the amino acids at 151-position et seq. are missing)

XXXXVII   des-Ser$^{153}$-IL-1β (IL-1β wherein Ser at 153-position is missing)

XXXXVIII  Met-[Gly$^4$]IL-1β (IL-1β having Met attached to its N terminal and in which Arg at 4-position is replaced by Gly)

In the following examples, physiological activities were determined by the following methods.

(1) Determination of IL-1 activity

Expressed in terms of LAF activity as measured by the method of J. J. Oppenhein et al. (J. Immunol., 116, 1466 (1976)) using thymus cells of a mouse of C3H/HeJ strain.

(2) Determination of GIF activity

Portions (0.1 ml) of the test solution diluted to varying concentrations were placed into the wells of 96-well microplate (Corning Co., Ltd.), 0.1 ml of Eagle's MEM suspension containing 10% FCS containing human melonoma cells A375 in an amount of 2 x 10$^4$ cells/ml was then placed into each well, and the cells were incubated in a CO$_2$ incubator (Napco Co., Ltd.) for 4 days. After the incubation, 0.05 ml of 0.05% Neutral Red (Wako Junyaku Co., Ltd.) was placed into each well, followed by incubation at 37°C for 2 hours. After removing the supernatant, 0.3 ml of phosphoric acid buffer saline was gently poured into each well for washing. After removing the washing, 0.1 ml of mixture of sodium phosphate monobasic and ethanol in equal amounts was placed into each well, the plate was shaken for several minutes by a micromixer, and the amount of pigment taken into the cell was measured at an absorbance of 540 μm using a photometer for 96-well microtitration plates (Titer check multiscane, Flow Lab.) to determine growth inhibition activity. The test group exhibiting 50% of the inhibition of cell growth of the control group, i.e., the test group which exhibited 1/2 the absorbance measured of the control group, was identified. The reciprocal of the number of times of dilution for the test group was taken as the GIF activity unit. Accordingly, when the GIF activity is 10 units, for example, the test solution, if diluted tenfold, still has activity to inhibit cell growth 50%.

The following drawings are referred to in reference examples and examples.

Fig. 1 is a restriction enzyme map of plasmid pGIF-α cDNA;

Fig. 2 is a diagram showing how plasmid ptrpGIF-α is constructed from plasmid pGIF-α and plasmid pTMI;

Figs. 3-a to 3-d are graphs showing the influence of anti-polypeptide I serum (neutralized antibody) on the GIF activity derived from plasmid pcD-GIG-16 or on the GIF activity derived from plasmid pcD-GIF-207;

Fig. 4 is a restriction enzyme map of cDNA of plasmid pcD-GIF-16 and of plasmid pcD-GIF-207;

Figs. 5 to 7 are graphs showing the results obtained by testing polypeptide I for its effect to promote production of CSF;

Fig. 8 is a graph showing the results obtained by testing polypeptide I for anti-arthritis effect;

Fig. 9 is a graph showing the results obtained by testing IL-1$\beta$ derivatives of the invention for CSF production promoting effect;

Fig. 10 is a graph showing the results obtained by testing IL-1$\beta$ derivatives of the invention for anti-inflammatory effect;

Fig. 11 is a graph showing the results obtained by testing IL-1$\beta$ derivatives of the invention for activity to prevent radiation sickness;

Fig. 12 is a graph showing the results obtained by testing IL-1$\beta$ derivatives of the invention for activity to prevent opportunistic infection;

Fig. 13 is a photograph showing the results obtained by testing the IL-1$\beta$ derivative of the invention for activity to induce and produce GM-CSF; and

Fig. 14 is a photograph showing the results obtained by testing the IL-1$\beta$ derivative of the invention for activity to induce and produce BSF-2.

Reference Example 1

Preparation of plasmid pGIF-$\alpha$

(1) Preparation of human lymphocytes

Human peripheral blood was collected, from which $1.9 \times 10^{10}$ lymphocytes were obtained by the Ficoll-Hypaque density gradient centrifugation method (Eur. J. Immunol. 4, 808 (1974)).

A quantity of the lymphocytes were suspended in RPMI 1640 medium containing 5% of human serum at a concentration of $4 \times 10^8$ cells/ml. The suspension was dividedly placed into Petri dishes, 9 cm in diameter, and the cells were incubated in the presence of 5% carbon dioxide gas at 37°C for one hour. The cells which were not adhered on the bottom of each dish were removed, and were stimulated with RPMI 1640 medium containing 10% FCS, 0.5 ng/ml of TPA (product of Sigma) and 10 $\mu$g/ml of LPS (product of Difco). After incubating the cells in 5% carbon dioxide gas at 37°C for 4 hours, $9 \times 10^8$ adhered lymphocytes were obtained using PBS and 0.02% EDTA.

(2) Preparation of mRNA

Human adhered lymphocytes ($9 \times 10^8$ cells) obtained by the procedure (1) were dissolved in 30 ml of 6M guanidine thiocyanate solution (6 M guanidine isothiocyanate, 5 mM sodium citrate (pH 7.0), 0.1 M 2-mercarpto ethanol and 0.5% Sarkosyl), and the DNA was thereafter sheared with a 50-ml syringe barrel having GI8G injection needle. A 12 g quantity of cesium chloride (CsCl) was completely dissolved in the solution. Portions (6.4 ml each) of the solution were superposed on 4 ml of 5.7 M CsCl (5.7 M CsCl-0.1M EDTA), and the combined layer was centrifuged by Beckman SW-40 Ti rotor at 31500 r.p.m. and 25°C for 20 hours. The RNA pellet sediment was washed with 70% ethanol and dissolved in TE solution (10 mM Tris-HCl (pH 7.5), 1 mM EDTA). To the solution were added 3M sodium acetate (pH 5.2) and ethanol in amounts of 1/9 and 2.2 times the amount of the solution, respectively, and the mixture was allowed to stand at -70°C for one hour. The mixture was then centrifuged at 15000 r.p.m. and 4°C for 20 minutes to collect the RNA, which was then dissolved in TE solution.

In this way, 250 $\mu$g of total RNA was prepared from about $9 \times 10^8$ adhered lymphocytes.

To obtain mRNA from the RNA, the RNA was subjected to column chromatography using oligo(dT)-cellulose (Collaborative Research Inc.). For adsorption, a solution of 10 mM Tris-HCl (pH 7.5), 0.5 M NaCl and 1 mM EDTA was used, and the column was washed with the same solution. The RNA was eluted with 10 mM Tris-HCl (pH 7.5) and 1 mM EDTA.

Consequently 17.5 $\mu$g of mRNA was obtained.

(3) Preparation of cDNA

From the mRNA obtained by the procedure (2), cDNA was synthesized in vitro, and recombinant DNA was prepared using Okayama-Berg plasmid vector (Okayama, H. and Berg, P., Mol. Cell. Biol., 2, 161 (1982)) and was transformed in E.coli to obtain cDNA library. The procedures given below were followed.

(3-1) Preparation of vector primer and linker DNA

DNA (400 $\mu$g) of pBR322-SV40 (0.71-0.86) was digested with 700 units of KpnI (NEB) at 37°C for 5 hours. The reaction was terminated with a mixture of 40 $\mu$l of 0.25 M EDTA (pH 8.0) and 20 $\mu$l of 10% SDS. The reaction mixture was subjected to extraction with the same volume of phenol-chloroform (1:1). DNA was precipitated using ethanol, followed by centrifugation and washing with 70% ethanol to collect DNA. The DNA obtained was dissolved in 200 $\mu$l of mixture of 140 mM sodium cacodylate, 30 mM Tris-HCl (pH 6.8), 1 mM $CoCl_2$, 0.1 mM DTT and 0.25 mM dTTP (containing 0.5 $\mu$Ci of $\alpha$-$^{32}$P-dTTP). The solution was treated with 400 units of terminal transferase (TTase, PL) for 30 minutes to elongate the dT chain. The reaction was terminated with 20 $\mu$l of 0.25 M EDTA and 10 $\mu$l of 10 % SDS, followed by extraction with phenol-chloroform 4 times. DNA was collected by precipitation with ethanol. Consequently, the dT chain was lengthened by about 70 bases.

The DNA thus obtained was digested at 37°C for 6 hours with 17 units of HpaI (NEB) and electrophoresed with agarose (low-melting agarose, BRL, 1%) to collect about 2.7 kb DNA fragment.

After the electrophoresis, the DNA was stained with 0.5 $\mu$g/ml of ethidium bromide, the agarose containing the approximately 2.7 kb fragment was cut out under UV irradiation, and to the agarose was added 5 times the volume of 20 mM Tris-HCl (pH 8.0)-1 mM EDTA to dissolve the agarose at 65°C over a period of 5 minutes, followed by extraction with phenol, then with phenol-chloroform (1:1) and thereafter with chloroform. The DNA was collected by precipitation with ethanol.

Subsequently, the vector primer DNA was purified by oligo(dA) cellulose column chromatography. The DNA was dissolved in 1 ml of 10 mM Tris-HCl (pH 7.3)-1 mM EDTA-1 M NaCl buffer. The solution was ice-cooled, then placed on the column equilibrated with the same buffer, which was thereafter washed with 1 ml of the same buffer and subsequently returned to room temperature. Elution then followed with 10 mM Tris-HCl (pH7.3)-1 mM EDTA to collect the peak fraction. The DNA was collected by precipitation with ethanol, then dissolved in 100 $\mu$l of 10 mM Tris-HCl (pH 7.3)-1 mM EDTA and stored at 4°C.

Linker DNA was prepared by the following procedure. pBR322-SV40 (0.19-0.32) DNA (100 $\mu$g) was digested with 120 units of PstI (NEB) at 37°C for 1.5 hours. The termination of the reaction was followed by extraction with phenol-chloroform and precipitation with ethanol. The DNA collected was dissolved in 50 $\mu$l of mixture of 140 mM sodium cacodylate, 30 mM Tris-HCl (pH 6.8), 1 mM $CoCl_2$, 0.1 mM DTT and 0.25 mM dGTP (containing 1 $\mu$Ci of $\alpha$-$^{32}$P-dGTP). The solution was acted on by 60 units of TTase for 20 minutes, whereby dG chain of 18 bases was attached. After the termination of the reaction, the DNA was collected, digested with 50 units of HindIII (Takara Shuzo Co., Ltd.) and electrophoresed with agarose (1.8%) in the same manner as above to collect about 0.28 kb DNA fragment and obtain 2.3 $\mu$g of linker DNA.

(3-2) Synthesis of cDNA and preparation of cDNA library

RNA (5 $\mu$g) was dried in a vacuo, then dissolved in 10 $\mu$l of 5 mM tris-HCl (pH 8.3) heated at 65°C for 5 minutes and immediately cooled to 37°C. To the reaction mixture was added 20 $\mu$l of mixture of 50 mM Tris-HCl (pH 8.3), 8 mM $MgCl_2$, 30 mM KCl, 0.3 mM DTT, 2 mM dNTP and 10 $\mu$Ci of $\alpha$-$^{32}$P-dCTP. The resulting mixture was maintained at 37°C for 5 minutes.

Ten units of RTase (reverse transcriptase, product of Seikagaku Kogyo Co., Ltd.) was added to the mixture and reacted therewith at 37°C for 15 minutes. With addition of 10 units of RTase again, the mixture was maintained at the same temperature for 15 minutes. The reaction was terminated with 2 $\mu$l of 0.25 mM EDTA (pH 8.0) and 1 $\mu$l of 10% SDS, followed by extraction with phenol-chloroform. To the extract were added 20 $\mu$l of 4M ammonium acetate and 80 $\mu$l of ethanol, and the mixture was frozen at -70°C for 15 minutes, then thawed at room temperature and centrifuged at 15000 r.p.m. and 4°C for 10 minutes. The sediment was dissolved in 20 $\mu$l of 10 mM Tris-HCl (pH 7.3). To the solution were added 19 $\mu$l of 4 M ammonium acetate and 80 $\mu$l of ethanol to reprecipitate.

The precipitate was collected, washed with 70% ethanol and dissolved in 15 $\mu$l of mixture of 140 mM sodium cacodylate, 30 mM Tris-HCl (pH 6.8), 1 mM $CoCl_2$, 0.1 mM DTT, 0.2 $\mu$g of poly A and 66 $\mu$M of ($\alpha$-$^{32}$P)dCTP (10 $\mu$Ci). TTase (P.L., 18 units) was added to the solution and reacted therewith at 37°C for 5 minutes. The reaction mixture was rapidly cooled to 0°C, and the reaction was terminated with 1.3 $\mu$l of 0.25 M EDTA and 0.65 $\mu$l of 10% SDS, followed by extraction with phenol-chloroform and precipitation with ethanol.

The precipitate was collected by centrifugation and then digested with 4 units of HindIII (Takara Shuzo Co., Ltd.) at 37°C for 2 hours. Termination of the reaction was followed by extraction with phenol-chloroform and precipitation with ethanol. The precipitate was collected and dissolved in 10 $\mu$l of mixture of

10 mM Tris-HCl (pH 7.3) and 1 mM EDTA. With addition of 3 $\mu$l of ethanol, the solution was preserved at -20°C.

One $\mu$l of the specimen thus obtained was maintained along with 5 ng of linker DNA in 10 $\mu$l of mixture of 10 mM tris-HCl (pH 7.5), 1 mM EDTA and 0.1 M NaCl at 65°C for 2 minutes and then at 42°C for 30 minutes, and thereafter cooled to 0°C. To the mixture was added 90 $\mu$l of a mixture solution of 20 mM Tris-HCl (pH 7.5), 4 mM MgCl$_2$, 10 mM (NE$_4$)$_2$SO$_4$, 0.1 M KCl, 0.1 mM $\beta$-NAD, 50 $\mu$g/ml BSA and 6 units/ml of E. coli DNA ligase, and then the combined solution was maintained at 12°C overnight.

To the solution were added 0.5 $\mu$l of 10 mM dNTP, 0.56 $\mu$l of 10 mM NAD, 0.5 $\mu$l of E. coli DNA polymerase I (product of Boehringer Mannheim) and 0.2 $\mu$l of RNase H (PL). The mixture was maintained at 12°C for one hour and then at 25°C for one hour, and thereafter frozen at -20°C for preservation.

E. coli HB101 strain was incubated to OD$_{550}$ of 0.45 in LB medium (10 g of bacto-trypton, 5 g of bacto-yeast extract and 10 g/liter of NaCl). The culture was ice-cooled for 5 minutes and then centrifuge at 4°C at 8000 r.p.m. for 5 minutes to harvest the cells. The pellets of cells were suspended in an ice-cooled mixture of 30 mM potassium acetate, 100 mM RbCl, 10 mM CaCl$_2$, 50 mM MnCl and 15% glycerin, maintained at 0°C for 5 minutes and centrifuged at 4°C at 8000 r.p.m. for 5 minutes. The cells collected were suspended again in a mixture of 10 mM MOPS (morpholinopropanesulfonic acid), 75 mM CaCl$_2$, 10 mM RbCl and 15% glycerin and maintained at 0°C for 15 minutes to prepare competent cells, which were thereafter preserved at -70°C.

The frozen suspension was thawed at room temperature. The DNA specimen (20 $\mu$l) was added to a 400 $\mu$l portion of the suspension, and the mixture was allowed to stand at 0°C for 30 minutes, subjected to heat shock at 42°C for 90 seconds and then allowed to stand again at 0°C for 1 to 2 minutes. With addition of 2 ml of LB medium, the mixture was maintained at 37°C for 30 minutes. LB medium (50 times the volume of the mixture) was inoculated with the mixture, followed by incubation at 37°C for 6 hours. Ampicillin was added to the culture to a concentration of 50 $\mu$g/ml, followed by incubation again overnight, whereby cDNA library was prepared. The cDNA library was preserved in 50% glycerin at -20°C.

(3-3) Preparation of synthetic probe

```
        Ala - Pro - Val - Arg - Ser - Leu - Asn - Cys -

  5' GCC   CCC   GTG   AGG   TCC   CTG   AAC   TGC

  3' CGG   GGG   CAC   TCC   AGG   GAC   TTG   ACG

     Thr - Leu - Arg - Asp - Ser - Gln - Gln - Lys -

     ACC   CTG   AGG   GAC   TCC   CAG   CAG   AAG

     TGG   GAC   TCC   CTG   AGG   GTC   GTC   TTC

     Ser - Leu - Val - Met

     TCC   CTG   GTG   ATG - 3'

     AGG   GAC   CAC   TAC - 5'
```

A nucleotide sequence (shown in the lowest line) complementary to the base sequence of the above formula is synthesized by the following method to use the complementary sequence as a probe for selecting a transformant having cDNA coding for IL-1$\beta$. Thus, the fully protected DNA was synthesized by the solid-phase phosphite triester method wherein N,N-dialkylmethyl phosphoramidite derivative is used as a condensation unit (Nature, 310, 105 (1984)), using an automatic synthesizer (380A DNA Synthesizer, Applied Biosystems Inc., Foster City, California 94404, U.S.A.). Subsequently, the fully protected DNA was treated with 28% ammonia water at 55°C for 10 hours, whereby the protective groups (i.e., the acyl groups for the amino groups of A, G and C) other than DMTr (dimethoxytrityl) group attached to the hydroxyl group at the 5' terminal were removed to obtain partially protected DNA. Next, the partially protected DNA was purified by reverse-phase HPLC using C$_{18}$ column and then treated with 80% acetic acid at room

17

temperature for 10 minutes to remove DMTr group. The nucleotide thus obtained was purified by electrophoresis using 10% polyacrylamide gel containing 7M urea and by Bio-gel P-30 (Bio-Rad Lab.) to obtain the desired DNA (60 mer).

The DNA (6 $\mu$g) thus obtained was reacted with 12 units of T4 polynucleotide kinase (Takara Shuzo) at 37°C for one hour in 50 $\mu$l of reaction mixture (50 mM tris-HCl (pH 7.6), 10 mM MgCl$_2$, 10 mM 2-mercapto ethanol, 0.2 mg/ml of fetal bovine thymus DNA and 50 $\mu$Ci ($\gamma^{32}$P)-ATP) to label the 5′ terminal of the DNA. To separate the labeled DNA from the unreacted $^{32}$P, the reaction mixture was subjected to column chromatography using Biogel P-30 (Bio-Rad). The fraction of the labeled DNA was precipitated with 3M sodium acetate in 1/9 of the volume of the fraction and ethanol in 2.5 times the volume thereof. The precipitate was collected by centrifugation, dissolved in 400 $\mu$l of mixture of 10 mM Tris-HCl (pH 8.0) and 1 mM EDTA and preserved at -20°C.

The specific activity of the resulting probe was at least $10^7$ cpm/$\mu$g DNA.

(3-4) Screening of cDNA library

Twenty-four nitrocellulose filters (Millipore HAIFO 8250), 80 mm in diameter, were placed over LB agar medium containing 50 $\mu$g/ml of ampicillin, and the cDNA library solution was spread over the filters, as so diluted that 5000 colonies were applied to each filter, followed by incubation overnight at 37°C.

A fresh nitrocellulose filter was placed over the filter where colonies appeared to prepare a replica filter.

The original filter (master filter) was preserved at 4°C. The replica filter was held on the same agar medium as above at 37°C for 6 hours for incubation and thereafter transferred onto LB agar medium containing 200 $\mu$g/ml of chloramphenicol, followed by incubation at 37°C overnight.

The filter was treated with 0.5 N NaOH, then with 1M Tris-HCl (pH 8.0) and thereafter with a mixture of 1 M Tris-HCl (pH 8.0) and 1.5 M NaCl. The filter was then dried in air and subsequently baked under a vacuum at 80°C for 2 hours.

The baked filter, while being lightly shaken, was maintained at 68°C overnight in 20 ml of solution of 1.2 M NaCl, 0.12 M trisodium citrate, 10 mg/ml of Ficoll, 10 mg/ml of polyvinylpyrrolidine, 10 mg/ml of BSA, 0.1% SDS and 1 mg/ml of salmon sperm DNA. The solution was replaced by a solution of 1.2 M NaCl, 0.12 M trisodium citrate, 10 mg/ml of Ficoll, 10 mg/ml of polyvinylpyrrolidine, 10 mg/ml of BSA, 0.1% SDS and $10^6$ cpm/ml of the probe, in which the filter was maintained at 42°C overnight with light shaking for hybridization.

After the hybridization, the filter was withdrawn from the solution, washed with a solution of 1.2 M NaCl, 0.12 M sodium citrate and 0.1% SDS three times at room temperature and thereafter washed with the same solution at 60°C until the count of the background of the filter became 200 cpm as determined by GM survey meter.

The filter was dried in air and then subjected to autoradiography at -70°C for 2 days using sensitized paper and x-ray film (Fuji RX).

After developing the film, the colonies present in the signal region were scraped off from the master filter, and the foregoing procedure was repeated to isolate the colonies with a positive signal.

Consequently, clone I-2 having a strong signal was isolated.

(3-5) Analysis of clone

Restriction enzyme map of plasmid pGIF-$\alpha$ cDNA contained in clone I-2 was prepared.

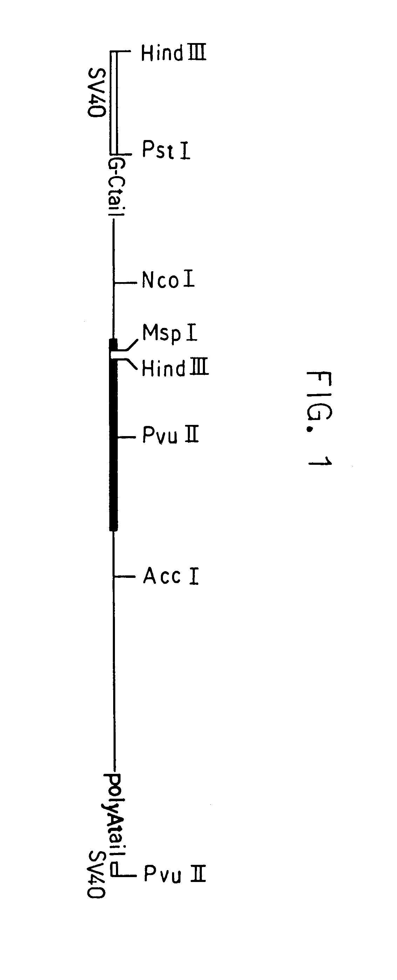

Fig. 1 shows the map.

Fig. 1 reveals that the cDNA has sites to be cleaved with NcoI (Nippon Gene), Hind III (Nippon Gene), PvuII (Nippon Gene) and AccI (Nippon Gene), one site by each, these cleavage sites being arranged in this order from the 5' terminus. It was found that the cDNA has a length of about 1.5 kb which is sufficient to code for IL-1$\beta$ having a molecular weight of about 18 kd.

Next, the base sequence of pGIF-$\alpha$ cDNA was determined by the Maxam-Gilbert chemical modification method (Meth. Enzym. 65, 499-566, 1980) and the dideoxynucleotide chain termination method using Ml3 phage (Messing, J. and Vieira, J., Gene, 19, 269-276 (1982)).

CTTATTACAGTGGCAATGAGGATGACTTG

TTCTTTGAAGCTGATGGCCCTAAACAGATGAAG
                                         Met  Lys

TGCTCCTTCCAGGACCTGGACCTCTGCCCTCTG
Cys  Ser  Phe  Gln  Asp  Leu  Asp  Leu  Cys  Pro  Leu

GATGGCGGCATCCAGCTACGAATCTCCGACCAC
Asp  Gly  Gly  Ile  Gln  Leu  Arg  Ile  Ser  Asp  His

CACTACAGCAAGGGCTTCAGGCAGGCCGCGTCA
His  Tyr  Ser  Lys  Gly  Phe  Arg  Gln  Ala  Ala  Ser

GTTGTTGTGGCCATGGACAAGCTGAGGAAGATG
Val  Val  Val  Ala  Met  Asp  Lys  Leu  Arg  Lys  Met

CTGGTTCCCTGCCCACAGACCTTCCAGGAGAAT
Leu  Val  Pro  Cys  Pro  Gln  Thr  Phe  Gln  Glu  Asn

GACCTGAGCACCTTCTTTCCCTTCATCTTTGAA
Asp  Leu  Ser  Thr  Phe  Phe  Pro  Phe  Ile  Phe  Glu

GAAGAACCTATCTTCTTCGACACATGGGATAAC
Glu  Glu  Pro  Ile  Phe  Phe  Asp  Thr  Trp  Asp  Asn

GAGGCTTATGTGCACGATGCACCTGTACGATCA
Glu  Ala  Tyr  Val  His  Asp  Ala  Pro  Val  Arg  Ser

CTGAACTGCACGCTCCGGGACTCACAGCAAAAA
Leu  Asn  Cys  Thr  Leu  Arg  Asp  Ser  Gln  Gln  Lys

AGCTTGGTGATGTCTGGTCCATATGAACTGAAA
Ser  Leu  Val  Met  Ser  Gly  Pro  Tyr  Glu  Leu  Lys

GCTCTCCACCTCCAGGGACAGGATATGGAGCAA
Ala Leu His Leu Gln Gly Gln Asp Met Glu Gln

CAAGTGGTGTTCTCCATGTCCTTTGTACAAGGA
Gln Val Val Phe Ser Met Ser Phe Val Gln Gly

GAAGAAGTAATGACAAAATACCTGTGGCCTTG
Glu Glu Ser Asn Asp Lys Ile Pro Val Ala Leu

GGCCTCAAGGAAAGAATCTGTACCTGTCCTGC
Gly Leu Lys Glu Lys Asn Lue Tyr Leu Ser Cys

GTGTTGAAAGATGATAAGCCCACTCTACAGCTG
Val Leu Lys Asp Asp Lys Pro Thr Leu Gln Leu

GAGAGTGTAGATCCCAAAAATTACCCAAAGAAG
Glu Ser Val Asp Pro Lys Asn Tyr Pro Lys Lys

AAGATGGAAAAGCGATTTGTCTTCAACAAGATA
Lys Met Glu Lys Arg Phe Val Phe Asn Lys Ile

GAAATCAATAACAAGCTGGAATTTGAGTCTGCC
Glu Ile Asn Asn Lys Leu Glu Phe Glu Ser Ala

CAGTTCCCCAACTGGTACATCAGCACCTCTCAA
Gln Phe Pro Asn Trp Tyr Ile Ser Thr Ser Gln

GCAGAAAACATGCCCGTCTTCCTGGGAGGGACC
Ala Glu Asn Met Pro Val Phe Leu Gly Gly Thr

AAAGGCGGCCAGGATATAACTGACTTCACCATG
Lys Gly Gly Gln Asp Ile Thr Asp Phe Thr Met

CAATTTGTGTCTTCCTAAAGAGAGCTGTACCCA
Gln Phe Val Ser Ser

GAGAGTCCTGTGCTGAATGTGGACTCAATCCCT

AGGGCTGGCAGAAAGGGAACAGAAGGTTTTTGA

GTACGGCTATAGCCTGGACTTTCCTGTTGTCTA

CACCAATGCCCAACTGCCTGCCTTAGGGTAGTG

CTAAGACGATCTCCTGTCCATCAGCCAGGACAG

TCAGCTCTCTCCTTTCAGGGCCAATCCCAGCCC

TTTTGTTGAGCCAGGCCTCTCTCTCACCTCTCC

TACTCACTTAAAGCCCGCCTGACAGAAACCAGG

CCACATTTTGGTTCTAAGAAACCCTCCTCTGTC

ATTCGCTCCCACATTCTGATGAGCAACCGCTTC

CCTATTTATTTATTTATTTGTTTGTTTGTTTTG

ATTCATTGGTCTAATTTATTCAAAGGGGGCAAG

AAGTAGCAGTGTCTGTAAAAGAGCCTACTTTTT

ATTAGCTATGGAATCAATTCAATTTGGACTGGT

GTGCTCTCTTTAAATCAAGTCCTTTAATTAAGA

CTGAAAATATATAAGCTCAGATTATTTAAATGG

GAATATTTATAAATGAGCAAATATCATACTGTT

CAATGGTTCTCAAATAAACTTCACTAAAAAAA

AAAAAAAAAAAAAAAAAAAAA

The formula shows that the underlined region of the 312nd to 371st nucleotides from the 5′ terminal is complementary to the synthesized probe. The nucleotide sequence had 75% homology with the nucleotide sequence determined according to the human codon usage frequencies.

Further when the cDNA of pGIF-α was searched for the longest reading frame, this frame was found to be the region of the 57th to 771st nucleotides from the 5′ terminal. This indicates that the cDNA of pGIF-α is cDNA coding for a human IL-1β precursor protein.

The transformant prepared by introducing the above plasmid, pGIF-α, into E. coli χI776 has been deposited under the name of Escherichia coli χI776/pGIF-α and the deposition number FERM BP-948 in Fermentation Research Institute, Agency of Industrial Science and Technology since December 12, 1985

Ref. Ex. 2 Preparation of Polypeptide I

(1) The following oligodeoxynucleotides (I) and (II) were prepared by the procedures described below.

5' HOCGATAATGGCTCCTGTACGTTCTCTGAACTGCACTCTCOH 3'    (I)
5' HOCGGAGAGTGCAGTTCAGAGAACGTACAGGAGCCATTATOH 3'    (II)

5'-O-Dimethoxytrityl and N-protected deoxynucleoside (Applied Biosystems Inc.) attached to macro-porous silica were used as starting materials. The nucleotide chain was successively lengthened from the 3' terminal toward the 5' terminal with the condensation units of 5'-0-dimethoxytrityl and N-protected deoxymononucleoside-3'-phosphoramidite, using an automatic synthesizer (380A DNA synthesizer, Applied Biosystems Inc.). The resulting product was treated with thiophenol for demethylation and further treated with 28% ammonia water at room temperature to remove the nucleotide from the silica, whereby fully protected oligonucleotide was obtained. The above procedure was executed entirely by the automatic synthesizer (Hunkapiller et al., Nature, 310, 105 (1984)).

The oligonucleotide was treated with 2 ml of 28% ammonia water at 55°C for 10 hours to remove the N-protective group and obtain 5'-O-dimethoxytrityloligonucleotide. One fifth of the amount of the product was purified by reverse-phase high-performance liquid chromatography using ODS (Yamamura Kagaku Kenkyusho Co., Ltd.) column and then treated with 150 μl of 80% acetic acid at room temperature for 20 minutes to obtain crude oligonucleotide. The product was further purified by reverse-phase high-performance liquid chromatography with ODS column to obtain the desired oligonucleotide.

The plasmid pGIF-α obtained by the procedure of Ref. Example 1, was cleaved with restriction enzymes AccI and ClaI to obtain a DNA fragment of about 1.2 kilo base pairs (kbp), which was isolated and purified by agarose gel electrophoresis. The DNA fragment was made blunt at the ends cleaved with restriction enzymes AccI and ClaI, using DNA polymerase I (Klenow fragment).

On the other hand, BamHI linker (5' HOCGGATCCGOH 3') was phosphorylated at the 5' terminal with T4 polynucleotide kinase and joined to the blunt-ended DNA fragment with T4 DNA ligase, followed by digestion with restriction enzyme BamHI and further with restriction enzyme MspI. The resulting reaction product was electrophoresed on agarose gel to isolate purified MspI-BamHI DNA fragment of about 540 bp.

The oligodeoxynucleotides (I) and (II) synthesized above were phosphorylated at the 5' terminal with T4 polynucleotide kinase and joined to MspI-BamHI DNA fragment using T4 DNA ligase, followed by digestion with restriction enzymes BamHI and ClaI. The reaction product was electrophoresed on agarose gel to isolate purified ClaI-BamHI DNA fragment of about 580 bp.

On the other hand, plasmid pTMI (Fumio Imamoto, Taishya, Vol. 22, 289 (1985)) was cleaved with restriction enzymes BamHI and ClaI, followed by agarose gel electrophoresis to isolate and purify DNA fragment of about 4.4 kbp having a trp promotor region. The DNA fragment and the ClaI-BamHI DNA fragment of about 580 bp prepared above were ligated with T4 DNA ligase to obtain the desired plasmid ptrpGIF-α for express polypeptide I.

The plasmid was introduced into E. coli HB101 for transformation, and the desired transformant E. coli HB101/ptrpGIF-α was selected by the restriction enzyme analysis of the plasmid DNAs which was obtained by the boiling method (T. Maniatis, E.F. Fritsch and J. Sambrook, Molecular Cloning, p. 366, Cold Spring Harbor Laboratory, (1982)).

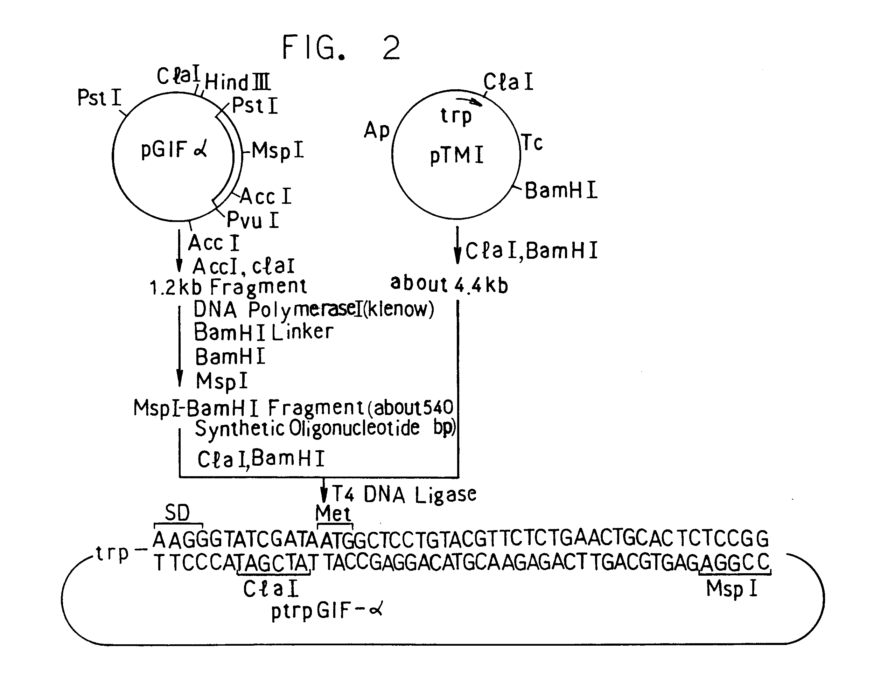

Fig. 2 schematically shows the above procedure.

The transformant prepared by introducing the plasmid ptrpGIF-α into E. coli χl776 has been deposited under the name of Escherichia coli χl776/ptrpGIF-α and deposition number FERM BP-949 in Fermentation Research Institute, Agency of Industrial Science and Technology since December 12, 1985.

(2) Culture of transformant

The transformant obtained above, i.e., E. coli HB101/ptrpGIF-α was incubated overnight at 37°C with shaking in 10 ml of LB medium (1% tryptone, 0.5% yeast extract and 0.5% NaCl) containing 50 $\mu$g/ml of ampicillin and 20 $\mu$g/ml of L-tryptophan. One-ml portion of the culture was inoculated in 50 ml of M9 minimum medium (0.6% $Na_2HPO_4$, 0.3% $KH_2PO_4$, 0.05% NaCl, 0.1% $NH_4Cl$, 2 mM $MgSO_4$, 0.2% glucose and 0.1 mM $CaCl_2$) containing 50 $\mu$g/ml of ampicillin and 1% Casamino acid and incubated at 37°C with shaking. The cells were harvested when the absorbance (O.D.) at 550 nm reached 1.0 and suspended in 5 ml of a solution of 15% sucrose, 50 mM Tris-HCl (pH 8.0) and 50 mM EDTA (pH 8.0). 500 $\mu$l of 10 mg/ml of lysozyme (as dissolved in 10 mM Tris-HCl (pH 8.0)) was added to the suspension, and 5 ml of a solution of 0.3% Triton X100, 187.5 mM EDTA (pH 8.0) and 150 mM Tris-HCl (pH 8.0) was further added. The mixture was allowed to stand at roof temperature for 15 minutes, then thoroughly stirred and centrifuged to obtain a GIF activity positive-supernatant of cell extract.

(3) Purification of Polypeptide I

Ion-exchange chromatography (CM-HPLC)

The cell extract supernatant prepared as above was dialyzed with 50 mM sodium acetate buffer (pH 5.5), and the dialyzate was subjected to ion-exchange chromatography (CM-HPLC) using Gilson high permeation liquid chromatography system (Gilson) under the following conditions.

| | |
|---|---|
| Column: | IEX-535CM (6.0 x 150 mm, Toyo Soda Co., Ltd.) |
| Eluent A: | 50 mM sodium acetate (pH 5.5) |
| Eluent B: | 50 mM sodium acetate (pH 5.5) containing 0.5 M Nacl |
| Flow rate: | 0.5 ml/min. |
| Fraction volume: | Retention time |
| | 0-60 min ...... 2 ml/4min/tube |
| | 60-120 min ... 0.5 ml/min/tube |
| | 120-180 min .. 2 ml/4 min/tube |

Concentration gradient:

| Time (min) | % B |
|---|---|
| 0 | 0 |
| 40 | 0 |
| 120 | 20 |
| 140 | 100 |
| 165 | 100 |
| 170 | 0 |

## Reverse-phase high-performance liquid chromotography

The CM-HPLC procedure resulted in a GIF active fraction with a retention time of 90 to 91 minutes.

The active fraction was then subjected to reverse-phase high-performance liquid chromotography under the following conditions.

| | |
|---|---|
| Column : | C$_4$ Highpore reverse-phase column (RP304, Bio-Rad, 250 mm x 4.6 mm (diam.) |
| Eluents : | A liquid = 0.1% TFA |
| | B liquid = acetonitrile-1% TFA (9:1) |
| Flow rate : | 1 ml/min. |
| Chart speed : | Retention time |
| | 0-50 min ..... 5 min/cm |
| | 50-80 min ... 2 min/cm |

Concentration gradient:

| Time (min) | % B |
|---|---|
| 0 | 0 |
| 5 | 0 |
| 15 | 20 |
| 75 | 45 |
| 80 | 100 |
| 85 | 100 |
| 90 | 0 |

Fraction volume:　　　　2 ml/2 min/tube

There was obtained at a retention time of 63.9 to 65.3 minutes the desired Polypeptide I exhibiting a single protein absorbance peak matching that of GIF activity.

The Polypeptide I thus obtained possesses IL-1 activity and the specific activity was 2.7 x 10$^7$ GIF units/mg protein.

## (4) Identification of Polypeptide I

## SDS polyacrylamide gel electrophoresis (SDS-PAGE)

The SDS-PAGE analysis of Polypeptide I obtained by the above procedure (3) was conducted in accordance with the method of Laemmli, U.K. (Nature, 277, 680 (1970)) under the following conditions

| | |
|---|---|
| Specimen: | The Polypeptide I fraction obtained by the reverse phase HPLC procedure was completely dried, then dissolved in Laemmli sample buffer (not containing 2-mercapto ethanol (2ME$^-$) or containing 2-mercapto ethanol in an amount of 1/20 the volume of the buffer (2ME$^+$)) and treated at 100°C for 4 minutes. |
| Gel : | 15% Polycrylamide gel 1.5 mm in thickness |
| Apparatus : | Protean, product of Bio-Rad |
| Electrophoresis : | 40 mA constant current for 2 hours |

The gel resulting from the electrophoresis was stained with Silver Stain Kit (Bio-Rad).

The results indicate that Polypeptide I is migrated as a single band at the position of about 17Kd in the case of 2ME$^+$ or of about 17.5Kd in the case of 2ME$^-$.

## Isoelectrofocussing (IEF)

Polypeptide I was subjected to IEF using PAG plate(LKB) 3.5 to 9.5 in pH range, and Model 1415 (Bio-Rad) under the following conditions.

| | |
|---|---|
| Specimen: | Four lanes were used, i.e. 0.037 μg, 0.074 μg and 0.74 μg portions of polypeptide I obtained by the foregoing procedure (3) and the following marker proteins (pI marker protein). |
| Marker proteins | |
| | Amyloglucosidase (3.50) |
| | Soybean trypsin inhibitor (4.55) |

24

β-lactoglobulin A (5.20)
Bovine carbonic anhydrase B (5.85)
Human carbonic anhydrase B (6.55)
Horse myoglobin-acidic band (6.85)
Horse myoglobin-basic band (7.35)
Lentil lectin-acidic band (8.15)
Lentil lectin-middle band (8.45)
Lentil lectin-basic band (8.65)
Tryspinogen (9.30)

Electrode solutions: Anode solution = 1M H$_3$PO$_4$ Cathode solution = 1M NaOH

Electrophoresis : With constant power of 1W/cm gel width with cooling (10°C) for 90 minutes

Staining: With silver stain Kit

The gel resulting from the electrophoresis was sliced at a spacing of 1 cm, subjected to extraction with use of 1 ml of distilled water with shaking (for two days) and then checked for pH to calculate the isoelectric point.

Polypeptide I had an isoelectric point (PI) of 6.8 ± 0.1 and appeared as a single band at this position.

Amino acid composition

The polypeptide I fraction (30 μl) obtained by the reverse-phase high-performance liquid chromotographic procedure (3) was carefully placed into the bottom of a thick-walled hard test tube made of Pyrex glass and 12 mm x 120 mm, and was dried in a vacuum in a desiccator containing sodium hydroxide pellets. A 50 μl quantity of 4N methanesulfonic acid (containing 0.2% 3-(2-aminoethyl)-indole and produced by Pierce) was added to the dry specimen within the tube. The interior of the tube was deaerated at 0.1 to 0.2 mm Hg for 1 minute and then sealed off. The specimen was hydrolyzed in a heater at 118°C over a period of 24 hours. After opening the tube, the mixture was neutralized with 46 μl of 4N sodium hydroxide and diluted to an amount of 450 μl with citric acid buffer.

A 250 μl quantity of the specimen solution was used for amino acid analysis by an amino acid analyzer (Model HITACHI 835, product of Hitachi Ltd.). The amino acids separated were detected by the o-phthalaldehyde method and quantitatively determined with reference to calibration curves prepared with use of authentic amino acids.

Table 1 shows the results in terms of the mole ratio of component amino acids based on Phe (9 moles). Under the above analysis conditions, Pro and Cys are not determinable. For Ser, Thr and Met, the percent recovery as achieved under the above conditions is given in parentheses.

## Table 1

| Amino acid | Mole ratio |
|---|---|
| Asp and/or Asn | 17.1 |
| Ser | 10.9 (80%) |
| Thr | 5.4 (90%) |
| Glu and/or Gln | 23.8 |
| Gly | 8.3 |
| Ala | 5.0 |
| Val | 10.8 |
| Met | 5.5 (90%) |
| Ile | 4.9 |
| Leu | 15.1 |
| Tyr | 3.9 |
| Phe | (9) |
| Lys | 14.9 |
| His | 0.9 |
| Trp | 0.8 |
| Arg | 3.0 |

Amino acid sequence

A 150 $\mu$l quantity of Polypeptide I fraction obtained by the reverse-phase high-performance chromatographic procedure (3) was analyzed by a protein sequencer (Applied Biosystems Inc.). Each resulting PTH-amino acid was suitably diluted with 100 to 50 $\mu$l of 33% aqueous acetonitrile solution, and a 5 $\mu$l portion of the dilution was injected into a chromatographic column by an autosampler,Waters 710B. For the chromatographic system, two pumps, Beckman Model 112, were operated by a controller, Model 421. The column used, measuring 2 mm x 250 mm and packed with Ultrasphere ODS-5 $\mu$m, was maintained at 55°C by a column heater. The flow rate was 0.3 ml/min. A mixture of 20 mM sodium acetate and acetonitrile was used for gradient elution. Absorbance was monitored at 269 nm. Analysis was conducted for 45 minutes.

The results of 20 cycles of analysis revealed that Polypeptide I obtaned by the procedure (3) had the following sequence of 20 amino acids at the N terminal.

Ala-Pro-Val-Arg-Ser-Leu-Asn-Cys-Thr-Leu-
Arg-Asp-Ser-Gln-Gln-Lys-Ser-Leu-Val-Met-

Ser was confirmed by one of by-products and was further confirmed as a dehydro form showing absorption at 322 nm. In cycle 8, the peak of by-product appeared to indicate Cys, and Cys was further identified by analyzing the original specimen after carboxamidemethylation.

Further polypeptide I was digested with trypsin to obtain peptide fragments. Amino acid composition analysis of the fragments revealed that the C terminal peptide was accurately contained in the polypeptide free of damage.

Stated more sepcifically, a 60 µg portion of polypeptide I was dissolved in 600 µl of 1% ammonium hydrogen carbonate. To the solution was added 20 µl of a solution of trypsin (0.2 mg/ml, product of Cooper Biomedical) in 1% ammonium hydrogen carbonate. The mixture was allowed to stand at 37°C for 24 hours to obtain peptides cleaved with trypsin. The peptide mixture was subjected to reverse-phase HPLC (C-18, 300Å, 4.6 x 150 mm; with use of 0.1% TFA as A solution and acetonitrile containing 1% TFA in 1/10 of the volume of the nitrile as B solution while increasing the amount of B solution at a rate of 1%/3 min for elution). All the components were isolated by the time the proportion of B solution increased to 40%. The peptides were subjected to amino acid analysis, whereby all the anticipated peptide fragments were identified. Especially, the C terminal peptide was found free from basic amino acids, and was found to contain the anticipated amino acids accurately by the analyses. Consequently, polypeptide I obtained by the procedure (3) had an accurate C terminal as anticipated.

In the same manner as above, the peptide fragments were checked for amino acid sequence. All the sequences confirmed were found to match the sequence of IL-1$\beta$. The confirmed sequences of the peptide fragments checked are shown below in amino acid numbers of IL-1$\beta$.

1-4, 5-11, 12-16, 17-27, 28-63, 64-65, 66-74, 75-88, 89-92, 95-98, 99-103, 104-109, 110-138, 139-153.

These results revealed that polypeptide I obtained by the procedure (3) was polypeptide I having the specified sequence. The calculated molecular weight of polypeptide I is 17376.59.

Example 1

(1) Preparation of Polypeptide VI

ptrpGIF-$\alpha$ obtained by the Ref. Ex. 2 was used for preparing Polypeptide VI having the amino acid sequence of polypeptide I wherein the 71st amino acid, Cys, from the N terminal was replaced by Ser, according to the site-specific mutagenesis method (Pro. Nat. Acad. Sci., 81, 5662-5666 (1984); Science 224, 1431 1984)).

Ml3 mp II phage vector was used as a single-strand DNA template. EcoRI/BamHI DNA fragment was isolated from plasmid ptrpGIF-$\alpha$ and cloned in Ml3 mp 11 phage at restriction enzyme EcoRI and BamHI sites to obtain single-strand (ss) DNA (Ml3-GIF-$\alpha$), which was then used as a mutagenesis template. Synthetic oligonucleotide [5'-CTGTCCTCAGTGTTG-3' (primer)] was phosphorylated with T4 polynucleotide kinase and hybridized with ss Ml3-GIF-$\alpha$DNA. The hybrid was annealed, thereafter treated with DNA polymerase I, (Klenow fragment) and T4 DNA ligase in the presence of dNTPs and incubated at 15°C for 18 hours.

The DNA obtained was introduced into JM105 competent cells for transformation. The resulting phage plaque (50 colonies) was inoculated onto an agar plate and incubated at 37°C for 18 hours. A filter containing the culture was treated with an alkali in the usual manner for denaturation, dried and then baked at 80°C for 2 hours. The DNA was prehybridized and then hybridized at room temperature with $^{32}$P probe prepared by labeling the primer with $^{32}$P-r-ATP. The filter resulting from the hybridization was washed with 6X SSC buffer at room temperature for 10 minutes and further at 37°C for 10 minutes, dried and thereafter subjected to autoradiography at -70°C for 18 hours.

Ml3-GIF-7ls was selected as a typical clone from among five mutant clones, infected with JM105 and incubated to prepare ssDNA and RF DNA.

Ml3 dideoxynucleotide sequencing was performed for ssDNA to confirm mutation of the contemplated gene.

Newly produced restriction enzyme DdeI site was also identified in RF DNA.

EcoRI/BamHI fragment was prepared from RF DNA produced in JM105, introduced into expression plasmid as in the above procedure (1) to obtain the desired polypeptide VI expression plasmid ptrpGIF-$\alpha$-7IS.

E. coli HB 101 transformant harboring the foregoing plasmid was deposited under FERM No.BP1296 as "Escherichia coli HB 101/ptrp GIF-$\alpha$-7IS" with Fermentation Research Institute, Agency of Industrial Science and Technology (FERM).

A cell extract supernatant was prepared with use of the plasmid in the same manner as in Ref. Example 2-(2) and checked for GIF activity. Consequently, the GIF activity was 2.4 x 10$^6$ units/ml culture when E. coli HB101 was used as the host.

The desired polypeptide VI was isolated from the cell extract supernatant and purified in the same manner as in Reference Example 2-(3).

(2) Preparation of polypeptide IV

Polypeptide IV was prepared in the same manner as in the above procedure (1) using plasmid ptrpGIF-α.

Ml3 mp ll phage vector was used as a single-strand DNA template. EcoRI/BamHI DNA fragment was isolated from plasmid ptrpGIF-α and cloned in Ml3 mp ll phage at restriction enzyme EcoRI and BamHI sites to obtain single-strand (ss) DNA (Ml3-GIF-α), which was then used as a mutagenesis template. Synthetic oligonucleotide [5'-CTGAACTCGACTCTC-3'(primer)] was phosphorylated with T4 polynucleotide kinase and hybridized with ss Ml3-GIF-αDNA. The hybrid was annealed, thereafter treated with DNA polymerase I, (Klenow fragment) and T4 DNA ligase in the presence of dNTPs and incubated at 15°C for 18 hours.

The DNA obtained was introduced into JM105 competent cells for transformation. The resulting phage plaque (100 colonies) was inoculated onto an agar plate and incubated at 37°C for 18 hours. A filter containing the culture was treated with an alkali in the usual manner for denaturation, dried and then baked at 80°C for 2 hours. The DNA was prehybridized and then hybridized at room temperature with $^{32}P$ probe prepared by labeling the primer with $^{32}P$-r-ATP. The filter resulting from the hybridization was washed with 6X SSC buffer at room temperature for 10 minutes and further at 37°C for 10 minutes, dried and thereafter subjected to autoradiography at -70°C for 18 hours.

Ml3-GIF-8s was selected as a typical clone from among four mutant clones, infected with JM105 and incubated to prepare ssDNA and RF DNA.

Ml3 dideoxynucleotide sequencing was performed for ssDNA to confirm mutation of the contemplated gene.

Newly produced restriction enzyme Hinfl site was also identified in RF DNA.

EcoRI/BamHI fragment was prepared from RF DNA produced in JM105, introduced into expression plasmid as in Reference Example 2-(1) to obtain the desired polypeptide IV expression plasmid ptrpGIF-α-8S.

A cell extract supernatant was prepared with use of the plasmid in the same manner as in Reference Example 2-(2) and checked for GIF activity. The activity was $1.2 \times 10^5$ units/ml culture when E. coli HB101 was used as the host, or $6.2 \times 10^5$ units/ml culture when E. coli W3110 was used.

From the cell extract supernatant, the desired polypeptide VI was isolated, followed by purification, by the same method as in Reference Example 2-(3).

(3) Preparation of polypeptide V

Plasmid ptrpGIF-α-7IS and ptrpGIF-α-8S obtained by the foregoing procedures were cleaved with restriction enzymes EcoRI and HindIII to isolate approximately 400 bp DNA fragment from ptrpGIF-α-8S and approximately 4600 bp DNA fragment from ptrpGIF-α-7IS. These fragments were ligated to obtain plasmid ptrpGIF-α-8S7IS for expressing the desired polypeptide V having the amino acid sequence of IL-1β - (polypeptide I) wherein the 8th and 71st amino acids, Cys, from the N terminal were each replaced by Ser.

In the same manner as in Reference Example 2-(2), a cell extract supernatant was prepared using the plasmid. (The GIF activity was $1.4 \times 10^4$ units/ml culture when E. coli HB101 was used as the host, or $5 \times 10^5$ units/ml culture when E. coli W3110 was used.) The desired polypeptide V was isolated from the cell extract supernatant and purified in the same manner as in Reference Example 2-(3).

(4) Preparation of polypeptide II

In the same manner as in the above procedure (1), polypeptide II expression plasmid ptrpGIF-α-4G was obtained using 5'-GCTCCTGTAGGTTCTCTG-3' as the primer.

E. coli HB 101 transformant harboring the foregoing plasmid was deposited under FERM No.BP1297 as "Escherichia coli HB 101/ptrp GIF-α-4G" with Fermentation Research Institute, Agency of Industrial Science and Technology (FERM).

In the same manner as in Reference Example 2-(2), a cell extract supernatant was prepared using the plasmid. (The GIF activity was $2.8 \times 10^5$ units/ml culture when E. coli HB101 was used as the host.) The desired polypeptide II was isolated from the cell extract supernatant and purified in the same manner as in Reference Example 2-(3).

In this purification procedure, polypeptide XXXXVIII was obtained as purified by rechromatography at the first half of the main peak of CM-HPLC. The polypeptide XXXXVIII was substantially equal to the polypeptide II in the activity to promote the production of CSF and was low in any of GIF activity, LAF activity and activity to promote the production of PGE.

(5) Preparation of polypeptide III

By the same procedure as the foregoing (1), polypeptide III expression plasmid ptrpGIF-$\alpha$-IIQ was obtained using 5$'$-GCACTCTCCAGGACTCACA-3$'$ as the primer.

In the same manner as in Reference Example 2-(2), a cell extract supernatant was prepared using the plasmid. (The GIF activity was 2.8 x $10^6$ units/ml culture when E. coli HB101 was used as the host.) The desired polypeptide III was isolated from the cell extract supernatant and purified in the same manner as in Reference Example 2-(3).

(6) Preparation of polypeptide VII

By the same procedure as the foregoing (1), polypeptide VII expression plasmid ptrpGIF-$\alpha$-102CT was obtained using 5$'$-TCTTCAACTAGATAGAA-3$'$ as the primer.

In the same manner as in Reference Example 2-(2), a cell extract supernatant was prepared using the plasmid. (The GIF activity was 7.4 x $10^4$ units/ml culture when E. coli HB101 was used as the host.) The desired polypeptide VII was isolated from the cell extract supernatant and purified in the same manner as in Reference Example 2-(3).

(7)Preparation of polypeptide VIII

By the same procedure as the foregoing (1), polypeptide VIII expression plasmid ptrpGIF-$\alpha$-140CT was obtained using 5$'$AAAGGCGGCTAGGATATAA-3$'$ as the primer.

In the same manner as in Reference Example 2-(2), a cell extract supernatant was prepared using the plasmid. (The GIF activity was 5.8 x $10^3$ units/ml culture when E. coli W3110 was used as the host.) The desired polypeptide VIII was isolated from the cell extract supernatant and purified in the same manner as in Reference Example 2-(3).

Further the plasmid ptrpGIF-$\alpha$-140CT was introduced into E. coli HB101 as the host for expression, giving a cell extract supernatant (1.3 x $10^6$ units/ml culture in GIF activity) in the same manner as above. Polypeptide I was isolated from the supernatant and purified similarly.

When E. coli HB101 is used as the host in the above case, supE acts as a nonsense suppressor, and UAG stop codon is read through as Gln to express polypeptide I. On the other hand, when E. coli W3110 is used as the host, UAG acts as the stop codon since supE gene is not coded for, with the result that polypeptide VIII is expressed which comprises a sequence of 140 amino acids.

(8) Identification of polypeptides I to VIII

IL-1$\beta$ derivatives (polypeptides I to VIII) of the invention prepared by the foregoing procedures (1) to (7) were subjected to SDS-PAGE (18% polyacrylamide gel, 2ME+) in the same manner as in Reference Example 2-(4).

Consequently, each of polypeptides I to VII migrated as a single band at the position of about 17.5 kd, and polypeptide VIII migrated similarly at the position of about 16 kd.

Further Western blotting (Proc. Natl. Acad. Sci., U.S.A., 76, 1420 (1979)) was conducted for polypeptides I to VIII using neutral antiserum for GIF activity of polypeptide I (prepared by immunizing a rabbit with polypeptide I obtained in Reference Example 2-(3) by the usual method), whereby each of these polypeptides was confirmed as a single band at the corresponding position.

Example 2

(1) The transformant (E. coli HB101/ptrpGIF-$\alpha$) obtained in Reference Example 2-(1) was incubated overnight at 37°C in 400 ml of LB medium containing 50 $\mu$g/mg of ampicillin and 20 $\mu$g/ml of L-tryptophan. M9 minimum medium (20 liters) containing 1% Casamino acid was inoculated with the culture (400 ml), followed by incubation at 37°C for 8.5 hours.

The resulting culture was centrifuged to collect the cells, which were suspended in 1M $Na_2HPO_4$ and allowed to stand overhight in a cool chamber. The suspension was thereafter dialyzed against 10 mM Tris-HCl buffer (pH 8.0) for 2 days.

The dialyzate obtained was centrifuged (10000 r.p.m., 30 minutes) to obtain a supernatant.

The supernatant was collected in an amount correspodning to 300 liters of E. coli culture, adjusted to a pH of 5.0 with 100 mM acetic acid and passed through Zeta Prep SP-250 cartridge (product of LKB) at a flow rate of 30 ml/min. The elution was conducted with 10 mM sodium acetate (pH 5.3) for 100 minutes, then with 50 mM sodium acetate (pH 5.5) containing 0.1 M NaCl for 220 minutes, and further with 50 mM sodium acetate (pH 5.5) containing 1M NaCl. The fractions were checked by HPLC to collect the fractions containing the desired substance, i.e. fractions No.8 to No.10 of 50 mM sodium acetate (pH 5.5) containing 0.1 M NaCl.

The combined fractions were adjusted to pH 4.5 with 100 mM acetic acid, subjected to HPLC (TSK Gel SP-5PW, 5.5 x 20 cm, product of Toyo Soda Mfg. Co., Ltd.) and to elution under the following conditions to obtain fractions of 65 to 72 minutes in retention time (r.t.).

Eluent A:      50 mM sodium acetate (pH 5.5)
Eluent B:      50 mM sodium acetate (pH 5.5) containing 0.5 M NaCl
Flow rate:      30 ml/min
Concentration gradient:

| Time (min) | % B |
|---|---|
| 0 | 0 |
| 30 | 0 |
| 84 | 9 |
| 104 | 100 |
| 124 | 100 |
| 134 | 0 |
| 180 | 0 |

Thus, polypeptide I was obtained as purified.

By ultrafiltration (YM-5 membrane), the product was concentrated to 20 mg/ml in the form of a solution of 20 mM sodium phosphate buffer (pH 7.0) by changing the buffer.

The concentrate was further passed through a filter apparatus equipped with Posidyne filter NFZ (product of Nohon Pall) for sterilization to obtain a pure product which was up to 100 pg/mg protein in pyrogen content.

The purification process afforded about 3 g of polypeptide I from 300 liters of E. coli culture.

(2) The fractions (r.t. 64 to 65 min) of the first half of the main peak of the above (1) was subjected to the chromatography again to obtain polypeptide X. $2.7 \times 10^6$ GIF units/mg protein.

Similarly, the fractions (r.t. 72 to 76 min) of the second half of the main peak gave polypeptide XI. $2.8 \times 10^7$ GIF units/ mg protein.

(3) Polypeptides I, X and XI obtained by the procedures (1) and (2) above were subjected to amino acid analysis in the same manner as in the Reference Example 2-(4).

Table 2 below shows the results.

The amino acid sequence of polypeptides X and XI was analyzed in the same manner as in Reference Example 2-(4) to identify the sequence at the N terminal thereof.

Table 2

| Amino acid | Mole rato | | |
|---|---|---|---|
| | Polypeptide I | Polypeptide X | Polypeptide XI |
| Asp and/or Asn | 17.7 | 17.5 | 16.4 |
| Ser | 13.0 | 13.0 | 12.9 |
| Thr | 5.8 | 5.7 | 5.7 |
| Glu and/or Gln | 24.0 | 24.0 | 23.0 |
| Gly | 8.6 | 8.5 | 8.5 |
| Ala | 5.2 | 5.2 | 4.1 |
| Val | 10.3 | 10.2 | 10.1 |
| Met | 5.8 | 6.7 | 5.8 |
| Ile | 4.9 | 4.9 | 4.8 |
| Leu | 15.5 | 15.6 | 15.6 |
| Tyr | 3.6 | 3.7 | 3.8 |
| Phe | (9) | (9) | (9) |
| Lys | 14.9 | 14.7 | 14.3 |
| His | 1.0 | 1.0 | 1.0 |
| Trp | present | present | present |
| Arg | 2.9 | 2.8 | 3.0 |

(4) Polypeptide I has two Cys residues (at the 8- and 71-positions) within the molecule. The state of SH group present on the side chain of these Cys residues was checked by the following method.

a. About 5 nmoles of polypeptide I obtained in Reference Example 2-(3) (in the form of 100 $\mu$l of solution of 50 mM sodium acetate (pH 5.5) containing 0.1 M NaCl) was placed into each of three test tubes A, B and C. A 300 $\mu$l quantity of 6 M guanidine hydrochloride solution containing 0.1 M Tris-HCl (pH 8.45) was further placed into each tube.

Next, 25 $\mu$l of 6 M guanidine hydrochloride solution was placed into each of the tubes A and B to prepare blanks. Into the test tube C was placed 25 $\mu$l of 6 M guanidine hydrochloride solution containing 2 $\mu$moles of dithiothreitol serving as a reducing agent. Nitrogen gas was introduced into each test tube for 1 minute, and the tubes were thereafter allowed to stand at 50°C for 2 hours.

Subsequently, 25 $\mu$l of 6 M guanidine hydrochloride solution was placed into the blank test tube A, and 25 $\mu$moles of 6 M guanidine chloride solution containing 4 $\mu$moles of iodoacetamide into each of the test tubes B and C. After introducing nitrogen gas into each test tube for 1 minute, the tubes were allowed to stand in the dark at 25°C for 30 minutes.

With addition of 5 $\mu$l of 10% TFA to the mixture in each of the test tubes A, B and C thus prepared, the mixture was subjected to reverse-phase HPLC ($C_3$) to purify the protein.

About 1/10 of the quantity of purified protein was placed into a test tube, dried, hydrolyzed with 4N methanesulfonic acid and checked for amino acid composition.

Consequently, nearly equal quantities of carboxymethylcysteine were detected from the test tubes B and C, whereas polypeptide I in the test tube A remained unchanged. This indicates that the polypeptide in the test tubes B and C was converted to S-carboxamidemethylated polypeptide I (since the polypeptide in the test tube B was not exposed to the reducing agent), therefore revealing that no disulfide (S-S) linkage was formed by the Cys residues in polypeptide I.

Further the product remaining in each of the test tubes A, B and C was dried and then dissolved in 600 $\mu$l of 1% ammonium bicarbonate solution. The solution was adjusted to a concentration of 1 $\mu$g/5 $\mu$l. A trypsin solution (7.5 $\mu$l) was added to the solution to digest the protein with the enzyme at 37°C for 20 hours.

The enzymatic reaction was terminated by addition of 10 $\mu$l of 10% TFA to the mixture, and peptide fragments were separated off by reverse-phase HPLC ($C_{18}$).

As a result, the test tubes B and C exhibited nearly the same pattern, while the test tube A showed a pattern different therefrom.

The peptides thus isolated were checked for amino acid composition in the same manner as above. The peptide fragment in the test tube A revealed the presence of peptide which would not occur in the absence of Cys.

These results demonstrated that no disulfide linkage was formed intramolecularly or inter-molecularly by the Cys residue of polypeptide.

b. The SH groups in the polypeptide I were quantitatively determined by the Ellman method (Arch. Biochem. Biophys., 82, 70 (1959)) in the following manner.

A 200 $\mu$g quantity (11.5 $\mu$moles) of polypeptide I obtained in Reference Example 2-(3) was dissolved in 1 ml of 0.1 M Tris-HCl buffer (pH 8.0) containing 6 M guanidine hydrochloride and 1 mM EDTA. On the other hand, fresh 0.05 M phosphate buffer (pH 7.0) was prepared which contained 0.01 M DTNB (5,5'-dithiobis(2-nitrobenzoic acid). (The buffer will hereinafter be referred to as the "Ellman buffer.")

One ml of 0.1 M Tris-HCl buffer (pH 8.0) containing 6N guanidine hydrochloride and 10 mM EDTA was placed into a control cell, and 1 ml of the above solution containing polypeptide I into a specimen cell. A 40 $\mu$l quantity of the Ellman reagent was admixed with the contents of each cell, whereupon the absorbance of the mixture was measured at 412 nm. After a maximum absorbance was achieved, the reduction in the absorbance due to the decomposition of DTNB was corrected to "0" in determining the concentration of SH groups.

The absorbance actually obtained at 412 nm was 0.328.

On the other hand, $\epsilon_{412}$ of 3-carboxylate-4-nitrothiophenolate ion in an aqueous solution containing 6 M guanidine hydrochloride was 13880 $M^{-1} \cdot cm^{-1}$ (Eur. J. Biochem., 30, 32 (1972)). This revealed that the SH groups in the polypeptide I solution were 0.0000245 in M/e and that 11.5 $\mu$moles of polypeptide I contained 24.5 $\mu$moles of SH groups.

The above result demonstrated that the Cys residues contained in polypeptide I had a free SH group.

Further little or no change was found in GIF activity and in the quantity of SH groups determined by the Ellman method when a solution of polypeptide I in water or in PBS (-) was repeatedly lyophilized 3 to 4 times.

(5) When a heterologeous protein is expressed in a large amount in E. coli or the like by a gene recombination technique, the protein is not infrequently accumulated as an inclusion body in E. coli cells. In such a case, collection of the protein from the cells requires a treatment under severe conditions which, for example, involve use of a denaturing agent such as 7 M guanidine hydrochloride, 8 M urea, 0.1% SDS, or the like. Such a treatment, however, is very likely to irreversibly cause damage to the desired protein including a higher structure thereof. Accordingly, it is a matter of great interest in gene recombination techniques to isolate the desired protein under mild conditions without using the denaturing agent to the greatest possible extent. In this respect, the method (1) described above is very favorable since the desired protein can be extracted and isolated under a very mild condition, i.e. by osmotic shock. The method is desirable also because the desired protein obtained has a higher structure resembling that of natural product.

(6) Using plasmid ptrpGIF-α, the polypeptide (IL-1β derivatives of the invention) listed in Table 3 below were prepared by site-specific mutagenesis in the same manner as in Example 1-(1). The polypeptides were expressed and purified and GIF activity thereof was measured by the above procedure (1), and SDS-PAGE was conducted in the same manner as in Reference Example 2-(4). In the examples to follow, like methods were used unless otherwise stated.

Table 3

| Polypeptide | Primer |
|---|---|
| XII | 5'-GAAAAGCTTTTTGTC-3' |
| XVII | 5'-TGGCTCCTGTATCTCTGAA-3' |
| XVIII | 5'-ACTGCACTCTCGACTCACA-3' |
| XIX | 5'-AGATGGAAAAGTTTGTCTT-3' |
| XX | 5'-CTCTGAACGCCACTCTCC-3' |
| XXI | 5'-GTTCTCTGAACACTCTCCG-3' |
| XXII | 5'-ACCTGTCCGCCGTGTTGA-3' |
| XXIII | 5'-ACCTGTCCGTCGTGTTGA-3' |
| XXIV | 5'-TGTACCTGTCCGTGTTGAA-3' |
| XXV | 5'-CCAAAGCTGAAGATG-3' |
| XXVI | 5'-ATATAACTTAATTCACCA-3' |
| XXVII | 5'-TCACCATGTAATTTGTGT-3' |
| XXVIII | 5'-TTCCCCAACCGGTACATC-3' |
| XXIX | 5'-AAAGCTCTCTACCTCCAG-3' |
| IX | 5'-CCCAAGTGGTAGATCAGCA-3' |
| XXXV | 5'-CCCAAGTGGTAGATCAGCA-3' |
| XXXIX | 5'-ATCGATAATGCGTTCTCT-3' |
| XXXX | 5'-ATCGATAATGAACTGCAC-3' |
| XXXXI | 5'-ATCGATAATGCTCCGGGA-3' |
| XXXXII | 5'-ATGGCTCCTCGTTCTCTG-3' |
| XXXXIII | 5'-CCTGTACGTCTGAACTGC-3' |
| XXXXIV | 5'-AAGATGGAACGATTTGTC-3' |
| XXXXV | 5'-GAAAAGCGAGTCTTCAAC-3' |
| XXXXVI | 5'-ATGCAATTTTAATCTTCCTA-3' |
| XXXXVII | 5'-TTGTGTCTTAATAAAGAG-3' |

| Polypeptide | Constructed plasmid | Host (E.coli) | M.W. (kd) | GIF Activity (units/ml culture) |
|---|---|---|---|---|
| XII | ptrpGIF-α-98L | W3110 | 17.5 | $2.0 \times 10^3$ |
| XVII | ptrpGIF-α-4R-d | HB101 | 17.4 | $7.3 \times 10^4$ |
| XVIII | ptrpGIG-α-11R-d | HB110 | 17.4 | $4.6 \times 10^2$ |
| XIX | ptrpGIF-α-98R-d | W3110 | 17.4 | $2.3 \times 10^3$ |
| XX | ptrpGIF-α-8A | HB101 | 17.5 | $2.4 \times 10^6$ |
| XXI | ptrpGIF-α-8C-d | HB101 | 17.4 | $1.6 \times 10^6$ |
| XXII | ptrpGIF-α-71A | HB101 | 17.5 | $1.6 \times 10^6$ |
| XXIII | ptrpGIF-α-71V | HB101 | 17.5 | $1.2 \times 10^6$ |
| XXIV | ptrpGIF-α-71C-d | HB101 | 17.4 | $3.5 \times 10^2$ |
| XXV | ptrpGIF-α-93L | HB101 | 17.5 | $5.5 \times 10^5$ |
| XXVI | ptrpGIF-α-144CT | HB101 | 16.5 | $2.6 \times 10^2$ |
| XXVII | ptrpGIF-α-148CT | HB101 | 16.9 | $2.4 \times 10^2$ |
| XXVIII | ptrpGIF-α-120R | HB101 | 17.5 | $8.5 \times 10^5$ |
| XXIX | ptrpGIF-α-30Y | HB101 | 17.5 | $3.2 \times 10^5$ |
| IX | ptrpGIF-α-121Q | HB110 | 17.5 | $3.2 \times 10^4$ |
| XXXV | ptrpGIF-α-121Q | W3110 | 13.7 | $3.9 \times 10^2$ |
| XXXXIX | ptrpGIF-α-NT3AA-d | HB101 | 17.4 | $1.2 \times 10^5$ |
| XXXX | ptrpGIF-α-NT6AA-d | HB101 | 16.8 | $9.4 \times 10^2$ |
| XXXXI | ptrpGIF-α-NT9AA-d | HB110 | 16.5 | $2.4 \times 10^2$ |
| XXXXII | ptrpGIF-α-3V-d | HB101 | 17.4 | $2.9 \times 10^5$ |
| XXXXIII | ptrpGIF-α-5S-d | HB101 | 17.4 | $8.4 \times 10^2$ |
| XXXXIV | ptrpGIF-α-97K-d | HB101 | 17.4 | $8.9 \times 10^2$ |
| XXXXV | ptrpGIF-α-99F-d | HB101 | 17.4 | $2.7 \times 10^2$ |
| XXXXVI | ptrpGIF-α-150CT | HB101 | 17.2 | $2.7 \times 10^5$ |
| XXXXVII | ptrpGIF-α-152CT | HB101 | 17.4 | $8.0 \times 10^5$ |

(7) Using plasmid ptrpGIF-α-102CT obtained in Example 1-(6) and E. coli W3110 as the host, polypeptide XXX, an IL-1β derivative of the invention, was prepared by similar expression and purification procedures. About 11.4 kd (by SDS-PAGE).

(8) Using plasmid ptrpGIF-α-4G and 5'-GCACTCTCCAGGACTCACA-3' as the primer, polypeptide XIV expression plasmid ptrpGIF-α-4GIIQ was prepared in the same manner as in the procedure (6).

Using the plasmid and E. coli HB101 as the host, polypeptide XIV, an IL-1β derivative of the invention, was obtained by similar expression and purification procedures. About 17.5 kd (by SDS-PAGE). $2.7 \times 10^5$ GIF units/ml culture.

(9) In the same manner as in Example 1-(3), polypeptide XV expression plasmid ptrpGIF-α-4G98L was prepared using plasmid ptrpGIF-α-4G and plasmid ptrpGIF-α-98L.

More specifically, the two plasmids were cleaved with restriction enzymes EcoRI and HindIII. Subsequently, an approximately 400 bp DNA fragment was cut out from ptrpGIF-α-4G, and an approximately 4.6 kbp DNA fragment from ptrpGIF-α-98L, and the two fragments were ligated.

Using plasmid ptrpGIF-α-4G98L and E. coli W3110 as the host, polypeptide XV, an IL-1β derivative of the invention, was obtained through similar expression and purification procedures. About 17.5 kd (by SDS-PAGE).

(10) Plasmid ptrpGIF-α-IIQ98L for expressing polypeptide XVI was prepared in the same manner as in the procedure (9) except that plasmid ptrpGIF-α-4G was replaced by plasmid ptrpGIF-α-IIQ (using approximately 400 bp DNA fragment thereof).

Polypeptide XVI, an IL-1β derivative of the invention, was obtained similarly using the plasmid. About 17.5 kd (by SDS-PAGE).

(11) Plasmid ptrpGIF-α-4GIIQ was used in place of plasmid ptrpGIF-α-4G in the procedure (9) to obtain about 400 bp DNA fragment from the plasmid. Plasmid ptrpGIF-α-4GIIQ98L for expressing polypeptide XIII was obtained similarly using the fragment.

Polypeptide XIII, an IL-1β derivative of the invention, was obtained similarly using this plasmid. About 17.5 kd (by SDS-PAGE).

(12) Plasmid ptrpGIF-α-8A7IS for expressing polypeptide XXXVI was prepared in the same manner as in Example 1-(3) using plasmid ptrpGIF-α-8A and plasmid ptrpGIF-α-7IS.

More specifically, the two plasmids were cleaved with restriction enzymes EcoRI and HindIII. Subsequently, an approximately 400 bp DNA fragment was cut out from ptrpGIF-α-8A, and an approxi-

mately 4.6 kbp DNA fragment from ptrpGIF-$\alpha$-7IS, and the two fragments were ligated.

Using plasmid ptrpGIF-$\alpha$-8A7IS and E. coli as the host, polypeptide XXXVI, an IL-1$\beta$ derivative of the invention, was obtained by similar expression and purification procedures. About 17.5 kd (by SDS-PAGE). 8.7 x $10^5$ GIF units/ml culture.

(13) Plasmid ptrpGIF-$\alpha$-8A7IA was prepared in the same manner as in the procedure (12) except that plasmid ptrpGIF-$\alpha$-7IA was used in place of plasmid ptrpGIF-$\alpha$-7IS (to use approximately 4.6 kbp DNA fragment thereof). E. coli HB 101 transformant harboring the foregoing plasmid was deposited under FERM No.BP1298 as "Escherichia coli HB 101/ptrp GIF-$\alpha$-8A7IA" with Fermentation Research Institute, Agency of Industrial Science and Technology (FERM).

Polypeptide XXXVII, an IL-1$\beta$ derivative of the invention, was similarly obtained using the plasmid. About 17.5 kd (by SDS-PAGE). 1.6 x $10^6$ GIF units/ml culture.

(14) Plasmid ptrpGIF-$\alpha$-7IV was used in place of plasmid ptrpGIF-$\alpha$-7IS in the procedure (12) to obtain about 4.6 kbp DNA fragment from the plasmid. Plasmid ptrpGIF-$\alpha$-8A7IV for expressing polypeptide XXXVIII was obtained similarly using the fragment.

Polypeptide XXXVIII, an IL-1$\beta$ derivative of the invention, was similarly obtained using this plasmid. About 17.5 kd (by SDS-PAGE). 2.1 x $10^6$ GIF units/ml culture.

(15) Polypeptides II to VIII and XXXI to XXXIV, IL-1$\beta$ derivatives of the invention, were prepared using the transformants of Example 1-(1) to (7) and Example 3-(6) to (9) to be given below, respectively, by the procedure (1).

These products migrated as a single band by SDS-PAGE. Polypeptides II to VIII migrated at the same position as above, while polypeptides XXXI to XXXXIV achieved the following results.

| Polypeptide XXXI | about 22 kd |
|---|---|
| Polypeptide XXXII | about 23 kd |
| Polypeptide XXXIII | about 27 kd |
| Polypeptide XXXIV | about 31 kd |

Example 3

(1) Incubation of U937 cells

Human histiocytic lymphoma U937 cells (Ascenso, J. L. et al., Blood, Vol. 57, p170 (1981)), 1.4 x $10^9$ in number, were placed into RPMI-1640 culture medium containing 25 ng/ml of 12-o-tetradecanoylphorbol-13-acetate (TPA, product of Pharmacia), 10 $\mu$g/ml of concanavalin A (ConA, product of Sigma) and 10% FCS to prepare a cell suspension having a concentration of 4 x $10^5$ cells/ml.

Ten-ml portions of the cell suspension were separately placed into dishes (Falcon 3003), 9 cm in diameter, and incubated in 5% carbon dioxide gas at 37°C for 3 days. After removing the culture supernatant by an aspirator, 10 ml of RPMI-1640 medium containing 10 $\mu$g/ml of bacterial lipopolysaccharide (LPS, product of Difco), 1 $\mu$g/ml of muramyldipeptide (MDP, product of Wako Junyaku Co., Ltd.) and 1 ng/ml of TPA was placed into each dish. The cells were incubated in this medium in 5% carbon dioxide gas at 37°C for 18 hours. The U937 cells adhering to the bottom of the dish were used for preparing mRNA.

(2) Preparation of mRNA

RNA was extracted by the combination of the guanidinium/hot phenol method (Feramisco, J. R. et al., J. Bio. Chem., Vol. 257, 11024 (1982)) and the guanidinium/caesium chloride method (Glisin, V, et al., Biochemistry, Vol. 13, 2633 (1974)).

To wash the U937 cells incubated by the procedure (1), the dishes were rinsed with 5 ml of PBS(-) solution after removing the supernatant. The cells were then dissolved with 1 ml of 4 M guanidine isothiocyanace solution (4 M guanidine isothiocyanate (product of Fluka), 50 mM Tris-HCl (pH 7.6), 10 mM EDTA and 2% sodium lauroyl sarkosinate placed into each dish. The solution was collected with rubber polisher and Pasteur pipette to obtain 420 ml of cell solution, which was maintained at 60°C and passed through 18G injection needle to shear the chromosome DNA. Subsequently, phenol heated to 60°C was added to the solution in an amount equal to that of the solution, and the mixture was stirred with 18G injection needle for further shearing. To the mixture were then added 210 ml of 0.1 M sodium acetate-10

mM Tris-HCl (pH 7.4)-1 mM EDTA solution and 420 ml of chloroformisoamyl alcohol mixture (24:1 in volume ratio). The resulting mixture was vigorously agitated at 60°C for 15 minutes, then ice-cooled and centrifuged at 3003 r.p.m. at 4°C for 20 minutes. To the aqueous layer collected was added ethanol in two times the amount of the aqueous layer. The mixture was allowed to stand overnight at -70°C to obtain crude RNA precipitate. The crude RNA was dissolved in 48 ml of 6M guanidine isothiocyarate-5 mM sodium citrate (pH 7.0)-0.1 M $\beta$-mercaptoethanol-0.5% sodium lauroyl sarkosinate solution. Caesium chloride (19.2 g) was then dissolved in the solution. Seven-ml portions of the resulting solution were then superposed on 4 ml of 5.7 M caesium chloride-0.1 M EDTA (pH 7.5). The mixture was centrifuged at 31500 r.p.m. at 25°C for 20 hours by Beckman SW40Ti rotor to collect RNA.

Thus, 9.7 mg of RNA was obtained.

To obtain mRNA from the RNA, the RNA was subjected to column chromatography using oligo(dT)-cellulose (product of Collaborative Research Inc.). For adsorption, 10 mM Tris-HCl (pH 7.5)-0.5 M NaCl-1 mM EDTA was used. For elution, 10 mM Tris-HCl (pH 7.5)-1 mM EDTA was used.

Consequently, 400 $\mu$g of mRNA was obtained.

(3) Preparation of cDNA library

cDNA library was prepared by the Okayama-Berg method by which cDNA can be expressed in animal cells. Thus, dT tail-attached vector primer for use in cDNA cloning was prepared from pcDVI, and dG tail-attached linker DNA from plasmid pLI, each by the method of Okayama et al. (Okayama, H. and P. Berg, Molecular and Cellular Biology, Vol. 3, p. 280 (1983)).

mRNA (15 $\mu$g) obtained by the procedure (2) was dissolved in 20 $\mu$l of 5 mM Tris-HCl (pH 7.5)-0.5 mM EDTA (pH 7.5) aqueous solution and incubated at 65°C for 5 minutes and then at 37°C for 5 minutes. The reaction mixture was adjusted to a total quantity of 40 $\mu$l containing 50 mM Tris-HCl (pH 8.3), 8 mM MgCl$_2$, 30 mM KCl, 0.3 mM dithiothreitol, 2 mM of each of dATP, dGTP, dCTP and dTTP, 2.8 $\mu$g of vector primer DNA, 60 units of RNase inhibitor (product of Promega Biotech) and 40 units of reverse transcriptase (product of Bio-Lad), followed by incubation at 37°C for 1 hour. The reaction was terminated by addition of 2 $\mu$l of 0.5 M EDTA (pH 7.5) and 2 $\mu$l of 10% SDS. Subsequently, the mixture was subjected to phenol-chloroform extraction and chloroform extraction, and the extract was precipitated from ethanol to collect vector primer cDNA:mRNA.

The collected vector primer cDNA:mRNA was incubated at 37°C for 5 minutes in 30 $\mu$l of a reaction mixture composed of 140 mM sodium cacodylate, 30 mM Tris-HCl (pH 6.8), 1 mM CaCl$_2$, 0.1 mM dithiothreitol, 0.3 $\mu$g of poly A, 66 $\mu$M dCTP and 38 units of terminal deoxynucleotidyl transferase (product of Pharmacia), whereupon 1.5 $\mu$l of 0.5 M EDTA (pH 7.5) and 1.5 $\mu$l of 10% SDS were added to the mixture to terminate the reaction. The mixture was subjected to phenol-chloroform extraction and chloroform extraction, and the extract was precipitated from ethanol to collect oligo dC tail-attached cDNA:mRNA-vector primer.

The collected nucleic acid was incubated at 37°C for 90 minutes in 20 $\mu$l of a reaction mixture comprising 7 mM Tris-HCl (pH 7.5), 7 mM MgCl$_2$, 60 mM NaCl, 100 $\mu$g/ml of bovine serum albumin and 12 units of restriction enzyme HindIII (product of Nippon Gene Co., Ltd.) Subsequently, 1 $\mu$l of 0.5 M EDTA (pH 7.5) and 1 $\mu$l of 10% SDS were added to the mixture to terminate the reaction, followed by extraction with phenol-chloroform and then with chloroform and by precipitation from ethanol to collect HindIII-decomposed oligo dC tail-attached cDNA:mRNA-vector primer. The product was dissolved in 10 $\mu$l of 10 mM Tris-HCl (pH 7.5)-1 mM EDTA (pH 7.5) (TE (pH 7.5)). One $\mu$l portion of the solution was incubated in 10 $\mu$l of a reaction mixture comprising the above TE (pH 7.5), 0.1 M NaCl and oligo dC tail-attached linker DNA (14 ng), first at 65°C for 2 minutes and then at 42°C for 30 minutes. The mixture was thereafter cooled to 0°C.

The reaction mixture was adjusted to 100 $\mu$l containing 20 mM Tris-HCl (pH 7.5), 4 mM MgCl$_2$, 10 mM (NH$_4$)$_2$SO$_4$, 0.1 M KCl, 50 $\mu$g/ml bovine serum albumin, 0.1 mM $\beta$-NAD (nicotinamide-adenine-dinucleotide, product of Pharmacia) and 0.6 $\mu$g of E. coli DNA ligase (product of Pharmacia) and incubated at 12°C overnight. To the reaction mixture were added 40 $\mu$M of each of dATP, dGTP, dCTP and dTTP and 0.15 mM of $\beta$-NAD. Further with addition of 0.4 $\mu$g of E. coli DNA ligase, 4.6 units of E. coli DNA polymerase I (product of Boehringer Mannheim) and 1 unit of E. coli RNase H (product of Pharmacia), the mixture was incubated at 12°C for 1 hour and then at 25°C for 1 hour.

E. coli HB101 was transformed using the reaction mixture thus obtained. The competent cells of this strain used were a product of Bethesda Research Laboratories (BRL). The cells were transformed according to BRL's manual.

Consequently, cDNA library was obtained which contained about 21000 clones.

EP 0 237 967 B1

(4) Transfection of monkey COS-1 cells

The cDNA library obtained by the above procedure (3) was divided into groups each including about 70 clones on the average. Plasmid DNA was prepared from each group.

The plasmid DNA was prepared by the alkaline lysis method (Molecular Cloning-A Laboratory Manual, Cold Spring Harbor Laboratory, 1982, p. 368).

Incubated monkey cells, COS-1 cells (Gluzman, Y., Cell, Vol. 23, p. 175 (1981)), were infected with the plasmid DNA thus prepared from each group for transfection. The transfection was conducted by the DEAE-dextran method (Yokota, T. et al., Proc. Natl. Acad. Sci. U.S.A., Vol. 81, p. 1070 (1984)). More specifically, the COS-1 cells treated with trypsin were suspended in RPMI-1640 medium containing 10% FCS and adjusted to a concentration of $1 \times 10^6$ cells/ml. Subsequently, 500 $\mu$l portions of the suspension were placed into 6 wells of a well plate each containing 2 ml of RPMI-1640 medium containing 10% FCS. After incubating the cells at 37°C overnight, the supernatant was removed, the cells were washed with a medium free from serum, and 1 ml of RPMI-1640 medium containing 10 $\mu$g/ml of plasmid DNA, 0.4 mg/ml of DEAE-dextran (product of Pharmacia), 50 mM Tris-HCl (pH 7.4) and 10% FCS was placed into each well, followed by incubation at 37°C for 4.5 hours. The supernatant was thereafter removed, the cells were washed with a medium free from serum and 2 ml of RPMI-1640 medium containing 150 $\mu$M chloroquine (product of Sigma Chemical Co.) and 10% FCS was placed into each well, further followed by incubation at 37°C for 3 hours. The supernatant was removed, and the cells were washed with a medium free from serum and thereafter incubated at 37°C for 72 hours with addition of 3 ml of RPMI-1640 medium containing 10% FCS to the cells in each well. After collecting the supernatant, 2 ml of RPMI-1640 medium containing 10% FCS was placed into each well, followed by two freeze-thaw cycles. The cell extract was then collected. The culture supernatant and the cell extract were checked for GIF activity.

The group exhibiting GIF activity was divided into 24 groups of 10 clones each, and the same procedure as above was repeated using these groups for tie determination of GIF activity. The same procedure as above was repeated for the clones within the group exhibiting GIF activity to identify the clone exhibiting GIF activity.

Consequently, two kinds of clones, i.e. pcD-GIF-16 and pcD-GIF-207, were obtained.

Table 4 shows the GIF activity (GIF units/ml) of these clones.

Table 4

| Clone | Culture supernatant | Cell extract |
|---|---|---|
| pcD-GIF-16 | 48.3 | 120.7 |
| pcD-GIF-207 | 62.6 | 196.9 |
| pcDVI (control) | 0 | 0 |

(5) Analysis of clones

To check whether the GIF-active substances derived from the two clones were identical, serially diluted antiserum for neutralizing the GIF activity of polypeptide I was added to each of the culture supernatant and cell extract of pcD-GIF-16 or pcD-GIF-207 which exhibited definite GIF activity, whereby the influence of the antiserum on the supernatant and the cell extract was determined.

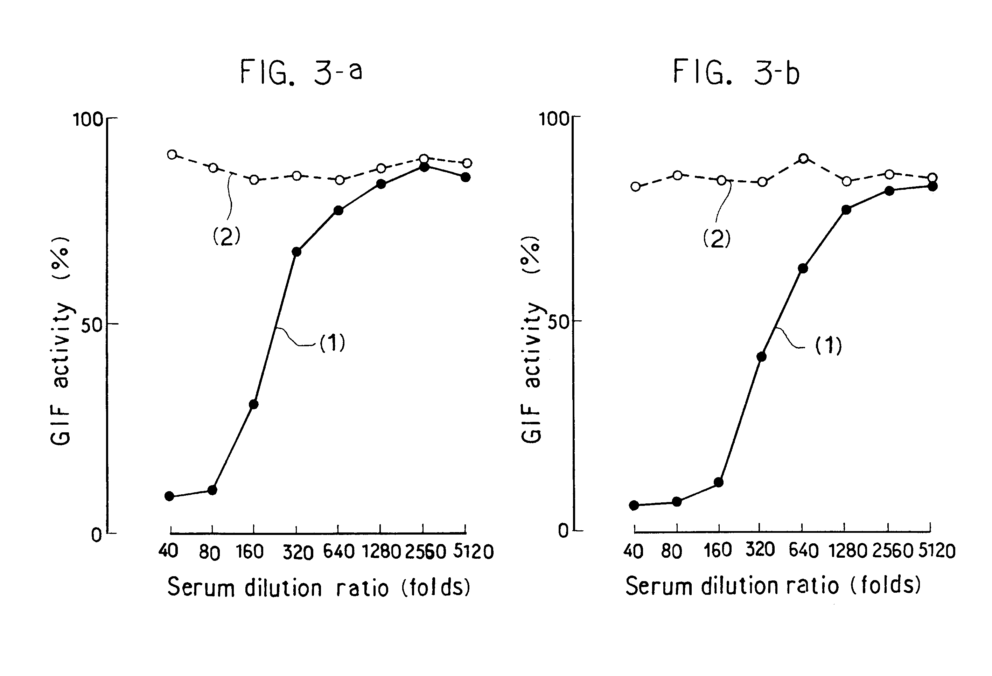

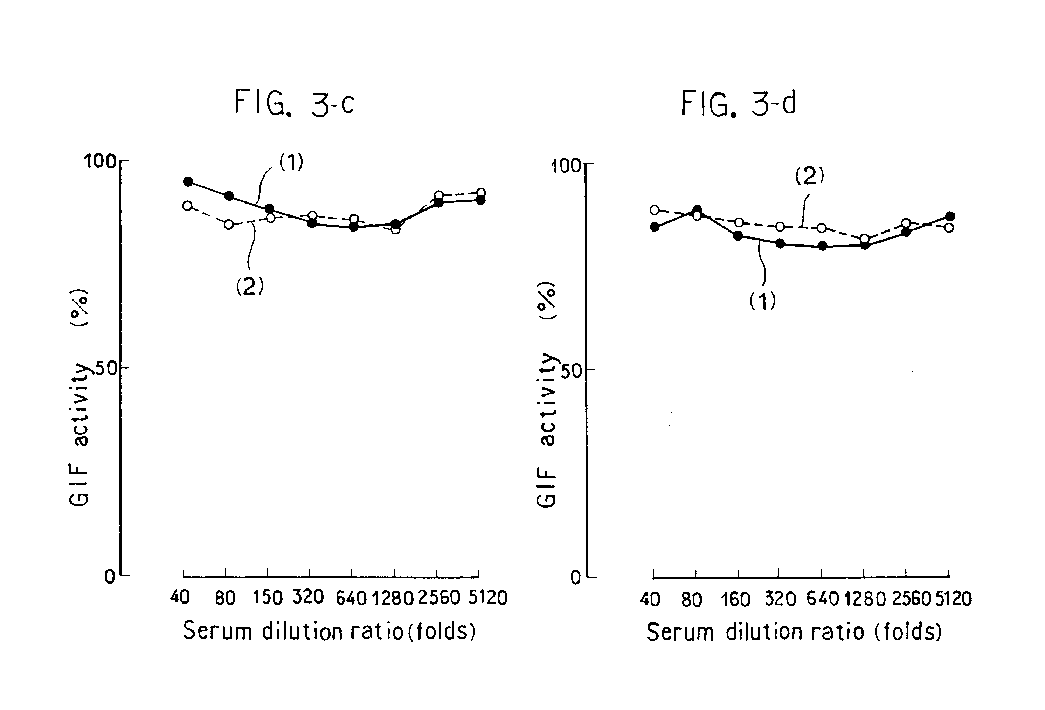

The results are shown in Fig. 3-a (pcD-GIF-16 culture supernatant), Fig. 3-b (pcD-GIF-16 cell extract), Fig. 3-c (pcD-GIF-207 culture supernatant) and Fig. 3-d (pcD-GIF-207 cell extract). In each of these diagrams, the serum dilution ratio (folds) is plotted as abscissa vs. the GIF activity (%) as ordinate, and (1) represents anti-polypeptide I serum and (2) represents normal serum.

As shown in Figs. 3-a and 3-b, the GIF activity derived from pcD-GIF-16 was completely neutralized by the addition of anti-polypeptide I serum concentration-dependently, whereas Figs. 3-c and 3-d reveal that GIF activity derived from pcD-GIF-207 was totally unaffected by the anti-polypeptide I serum. This indicates that the GIF-active substance from pcD-GIF-207 immunologically differs from polypeptide I and the GIF-active substance derived from pcD-GIF-16.

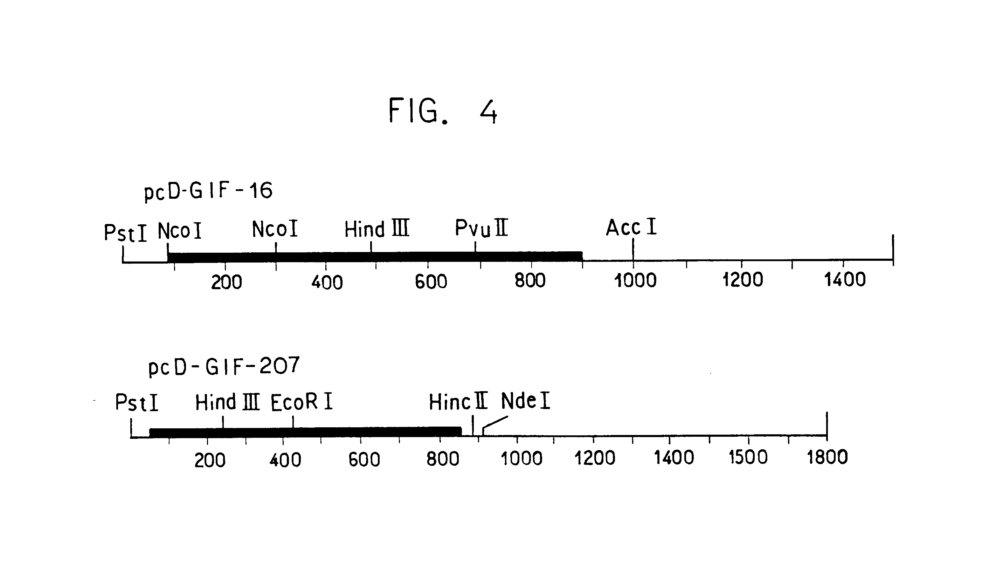

Fig. 4 shows a cDNA restriction enzyme map of plasmid pcD-GIF-16 and that of plasmid pcD-GIF-207. The cDNA base sequence of these plasmids was determined by the base-specific chemical modification method (Methods in Enzymology, Vol, 65, p. 499 (1980)) and by dideoxy chain termination (Proc. Natl. Acad. Sci., U.S.A., Vol. 74, p. 5463 (1977)) using phage Ml3 vector (Gene, Vol, 19, p. 269 (1982)).

37

The results established that the cDNA possessed by pcD-GIF-16 was identical with the cDNA sequence in the coding region of IL-1β as determined by March et al. and that cDNA of pcD-GIF-207 was identical with the cDNA sequence in the coding region of IL-1α (Carl J. March et al., Nature, Vol, 315, p. 641 (1985)).

(6) Preparation of polypeptide XXXIV

The cDNA clone pcD-GIF-16 of IL-1β almost of full length and obtained by the procedure (5) was cleaved with restriction enzyme PvuII, further partially cleaved with restriction enzyme NcoI and electrophoresed on agarose gel to isolate and purify 380 bp NcoI-PvuII DNA fragment.

Mung bean nuclease was caused to act on the DNA fragment, whereby the cohesive end produced by cleavage with restriction enzyme NcoI was smoothened.

Next, the 5' terminals of synthetic oligonucleotides (5'-GATAATG-3' and 5'-CATTAT-3') were phosporylated with T4 polynucleotide kinase and ligated with the DNA fragment with T4 DNA ligase. The ligated block was cleaved with restriction enzymes ClaI and HindIII and then electrophoresed on agarose gel, giving about 400 bp ClaI-HindIII DNA fragment A as isolated and purified.

On the other hand, expression plasmid ptrpGIF-α previously prepared was cleaved with restriction enzymes ClaI and HindIII. DNA fragment B, which was the larger, was isolated and purified by agarose gal electrophoresis.

The fragments A and B were ligated with T4 DNA ligase, and the ligated block was transformed into E. coli HB101.

The plasmid DNA of the resulting transformants was extracted, and the cleavage mapping with restriction enzymes was analyzed to select the desired transformant.

The desired transformant thus isolated (E. coli HB101/ptrpGIF-α-V) was incubated overnight with shaking at 37°C in 10 ml of LB medium containing 50 μg/ml of L-tryptophan. M9 minimum medium (50 ml) containing 50 μg/ml of ampicillin and 1% Casamino acid was inoculated with 1 ml of the culture, followed by incubation at 37°C with shaking. When the absorbance of the culture reached about 1.0 at 550 nm, the cells were collected and suspended in 5 ml of 15% sucrose-50 mM Tris-HCl-50 mM EDTA (pH 8.0). To the suspension were added 500 μl of solution of 10 mg/ml of lysozyme (as dissolved in 10 mM Tris-HCl (pH 8.0)) and 5 ml of 0.3% Triton X100-187.5 mM EDTA (pH 7.0)-150 mM Tris-HCl (pH 8.0) solution. The mixture was allowed to stand at room temperature for 15 minutes, then sonicated and centrifuged to obtain a supernatant of cell extract.

The GIF activity of the supernatant was found to be 112 units/ml culture.

(7) Preparation of polypeptide XXXII

Plasmid pGIF-α obtained previously was cleaved with restriction enzymes NcoI and AccI and electrophoresed on agarose gel to isolate and purify about 0.7 kb NcoI-AccI DNA fragment, which was treated with DNA polymerase I (Klenow fragment) to smoothen its opposite ends.

On the other hand, plasmid pTMI was cleaved with restriction enzyme HindIII and treated with DNA polymerase I (Klenow fragment) to smoothen the cut ends.

The two DNA fragments were ligated with T4 DNA ligase, and the ligated block was transformed into E. coli HB101. Plasmid DNA was extracted from the transformants by the boiling method. The desired transformant was selected with reference to the restriction enzyme map of the extract.

The transformant (E. coli HB101/ptrpGIF-α-III) thus isolated was incubated in the same manner as E. coli HB101/ptrpGIF-α-V in the foregoing procedure (6) to obtain a cell extract supernatant. The GIF activity of this supernatant was 190 units/ml culture.

(8) Preparation of polypeptide XXXIII

The plasmid for expressing this polypeptide was prepared by removing unnecessary portions of base sequence by site-specific mutagenesis as will be described in detail below.

pGIF-α was cleaved with restriction enzymes PstI and AccI and then electrophoresed on agarose gel to isolate and purify about 0.9 kb PstI-AccI DNA fragment, which was treated with T4 DNA polymerase to smoothen its opposite ends.

Using T4 DNA ligase, this fragment was ligated to a DNA fragment separately prepared from plasmid pTMI by cleavage with restriction enzyme HindIII and treatment with DNA polymerase (Klenow fragment) to provide a smooth cut end. The ligated block was transformed into E. coli HB101. The desired transformant was selected with reference to the restrction enzyme map of plasmid DNA extracted by the boiling method.

The DNA base sequence of the plasmid obtained was also identified. This plasmid will hereinafter be referred to as "pTMI-I-2".

Next, plasmid pTMI-I-2 was cleaved with restriction enzymes EcoRI and HindIII and electrophoresed on agarose gel to isolate and purify about 740 bp EcoRI-HindIII DNA fragment, which was then ligated to EcoRI and HindIII restriction enzyme sites of MI3mpII phage (RF) using T4 DNA ligase. Single-stranded DNA (mpII-trp-I-2(E/M)) was obtained from the product for use as a template for mutagenesis.

Synthetic oligonucleotide (5'-CACGTAAAAAGGGTATCGATAATGAAGTGCTCCT-3' (primer)) was phosphorylated with T4 polynucleotide kinase and hybridized with ss mpII-trp-I-2(E/M). The hybrid was annealed, treated with DNA polymerase I (Klenow fragment) and with T4 DNA ligase in the presence of dNTPs, and incubated at 15°C for 2 days.

The DNA obtained was transformed into JM105 competent cells and an agar plate was inoculated with 200 of the resulting colonies, followed by incubation at 37°C for 18 hours. A filter containing the grown colonies was treated with an alkali in the usual manner for denaturation, dried and then baked at 80°C for 2 hours. The filter was hybridized and further hybridized at room temperature with $^{32}$P-probe prepared by labeling the primer with $^{32}$P-$\gamma$-ATP at its 5' end. The resulting filter was washed with 6 x SSC buffer at room temperature for 10 minutes and then with 0.25 x SSC buffer at 60°C for 5 minutes, dried and thereafter autoradiographed at -70°C for 18 hours.

mpII-trp GIF-$\alpha$-IV(E/M) was selected from among the mutant clones as a typical example, infected with JM105 and incubated to prepare ss DNA and RF DNA.

MI3 dideoxy chain termination sequencing of the resulting ss DNA showed that the desired gene had been deleted.

Further about 630 bp EcoRI-HindIII DNA fragment was isolated from the resulting RF DNA and purified by agarose gel electrophoresis.

On the other hand, about 4.2 kb EcoRI-HindIII DNA fragment was similarly isolated from plasmid pTMI-I-2 and purified, and was ligated with the above fragment, i.e. approximately 630 bp EcoRI-HindIII DNA fragment, using T4 DNA ligase. The ligated block was transformed into E. coli HB101.

The desired transformant was selected with reference to the restriction enzyme map of plasmids extracted by the boiling method from the transformants obtained.

The transformant (E. coli HB101/ptrpGIF-$\alpha$-IV) thus isolated was incubated in the same manner as E. coli HB101/ptrpGIF-$\alpha$-V in the procedure (6) to obtain a cell extract supernatant , which was found to have GIF activity of 336 units/ml culture.

(9) Preparation of polypeptide XXXXI

In the same manner as in the procedure (8), a transformant harboring plasmid ptrpGIF-$\alpha$-II for expressing the desired polypeptide was prepared.

The template used was the same as ss DNAmpII-trp-I-2(E/M) used above. The primer used was synthetic oligonucleotide (5'-CACGTAAAAAGGGTATCGATAATGCTGGTTCCCT-3').

The plasmid-harboring transformant (E. coli HB101/ptrpGIF-$\alpha$-II) was similarly incubated to obtain a cell extract supernatant, which was found to have GIF activity of 112 units/ml culture.

While the GIF activity of IL-1$\beta$ (polypeptide I) of the invention and the derivatives thereof according to the invention has been described, the following tests were conducted.

Table 5 shows the LAF activity (U) per mg of polypeptides, calculated as protein of polypeptide I, as determined by the RIF method using the aforementioned antiserum against polypeptide I.

Table 5

| Polypeptide | LAF activity (U/mg) |
|---|---|
| Polypeptide I | $4.5 \times 10^6$ |
| Polypeptide II | $1.6 \times 10^5$ |
| Polypeptide III | $6.2 \times 10^6$ |
| Polypeptide IV | $2.6 \times 10^5$ |
| Polypeptide V | $2.1 \times 10^5$ |
| Polypeptide VI | $1.3 \times 10^7$ |
| Polypeptide VII | $8.0 \times 10^3$ |

Pharmacological Test Example 1

Testing polypeptide I for effect to induce CSF production

(1) Test of inducing effect on CSF production by human lung cells

The following test was conducted using human embryonic lung fibroblasts (HFL-1, ATCC registered cell strain No.CCL-153) which produce CSF.

HFL-1 cells were suspended in Ham's F-12K culture medium, (Ham, R.G., Proc. Natl. Acad. Sci., 53, 288 (1965) containing 10% FCS to a concentration of $2 \times 10^5$ cells/ml.

Polypeptide I obtained in the foregoing reference example was added to portions of the cell suspension in varying concentrations. Each of the mixtures was incubated in a carbon dioxide gas incubator at 37°C for 24, 48 or 72 hours. The culture supernatant was collected, and the amount of CSF produced and accumulated in the supernatant was measured using mouse bone marrow cells (Lewis, I.C. et al, J. Immunol., 128, 168 (1982)).

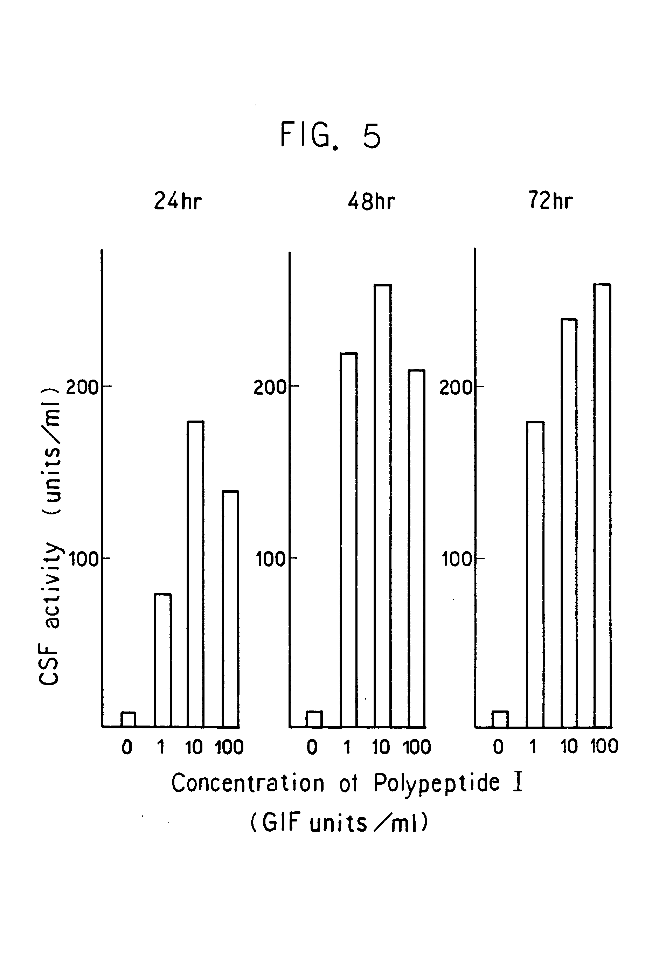

Fig. 5 shows the results achieved by polypeptide I during different periods of incubation. In the diagram, the concentration (GIF units/ml) of polypeptide I is plotted as abscissa vs. the CSF activity (units/ml) as ordinate.

The results achieved reveal that the addition of polypeptide I increases the amount of CSF produced by HFL-1 cell strain to as many as hundreds of times the amount produced in the absence of the polypeptide.

(2) Test of inducing effect of polypeptide I on CSF production by cells derived from human skin

The following test was conducted using normal human skin-derived cell strain, CRT-1445 (ATCC No.)

Cells of the above strain were suspended in Dulbecco MEM culture medium (Dulbecco, R. and Freeman, G., Virology, 8, 396 (1959)) containing 10% FCS to a concentration of $2 \times 10^5$ cells/ml.

Polypeptide I obtained in the foregoing reference example was added to portions of the cell suspension in varying concentrations. Each of the mixtures was incubated in a carbon dioxide gas incubator at 37°C for 24, 48 or 72 hours. The culture supernatant was collected, and the amount of CSF produced and accumulated in the supernatant was measured using mouse bone marrow cells as in the test (1).

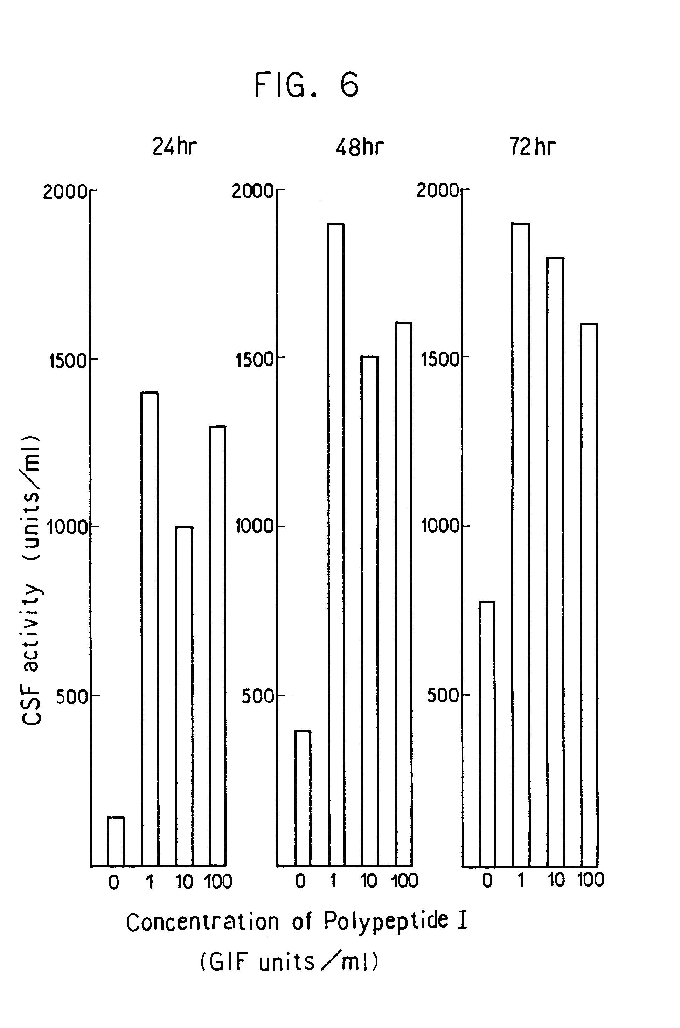

Fig. 6, like Fig. 5, shows the results achieved.

Fig. 6 shows that the addition of polypeptide in an amount of at least one unit/ml calculated as GIF activity to fibroblasts derived from the normal human skin gives the cells remarkably enhanced ability to produce CSF.

(3) Test of inducing effect on CSF production in vivo

The following animal experiment was conducted to substantiate that polypeptide I, when administered to the animal, acts to induce the production of CSF in vivo.

To normal mice (BALB/c strain, purchased from Experimental Animal Cooperative Association off Shizuoka Prefecture, Japan) was intravenously given polypeptide I obtained in Reference Example 2 in varying amounts ($10^3$ to $10^5$ units/animal calculated as GIF activity). Blood was collected from the animal 2, 4, 8, 12 and 24 hours after the administration and checked for the concentration of CSF in the serum using mouse bone marrow cells.

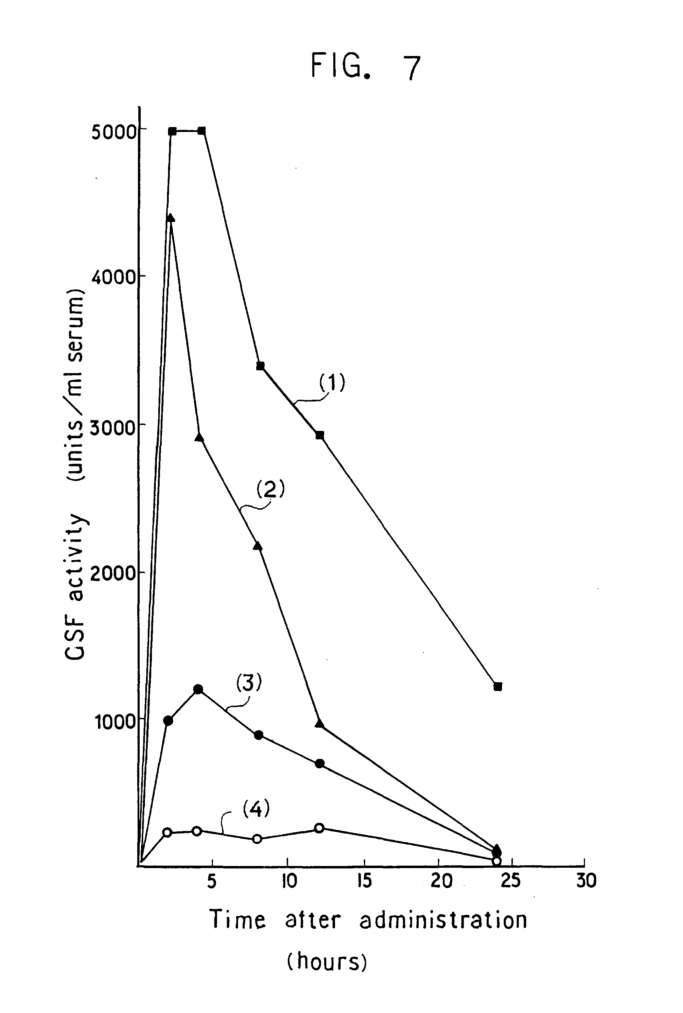

The results are given in Fig. 7, in which the time (hours) elapsed after the administration is plotted as abscissa vs. the CSF activity (units/ml serum) as ordinate. In the diagram, (1) represents a group to which polypeptide I was given at a dose of 100,000 GIF units/animal, (2) represents a group with a dose of 10,000 GIF units/animal, (3) represents a group with a dose of 1,000 GIF units/animal, and (4) represents a control group to which HSA was given at a dose of 10 $\mu$g/animal.

Fig. 7 reveals that polypeptide I, when given to animals, remarkably increases the CSF concentration of the serum of the animal. It is seen that polypeptide I greatly promotes the production of CSF in vivo in proportion to the dose.

Pharmacological Test Example 2

Rats with adjuvant arthritis were prepared by the method of Pearson (Pearson, C. M., Proc. Soc. Exp. Biol. Med., 91, 95 (1956)), Ward and Jones (Ward, J. R. and Jones, R. S., Arthritis Rheumatism, 5, 557 (1962)). More specifically, 0.05 ml of an adjuvant prepared by suspending dead cells of Mycobacterium butyricum in fluid paraffin was intracuteneously injected into the base portion of the tail of each of male rats of S.D. strain. On the 14th day, the animals were grouped (n = 6) according to the degree of swelling of the foot. During the period of the following 5 days, polypeptide I obtained in the reference example or the solvent therefor (saline for the control group) was intracuteneously given to the animals. The volume of the foot was measured from day to day to evaluate the influence of the polypeptide on arthritis.

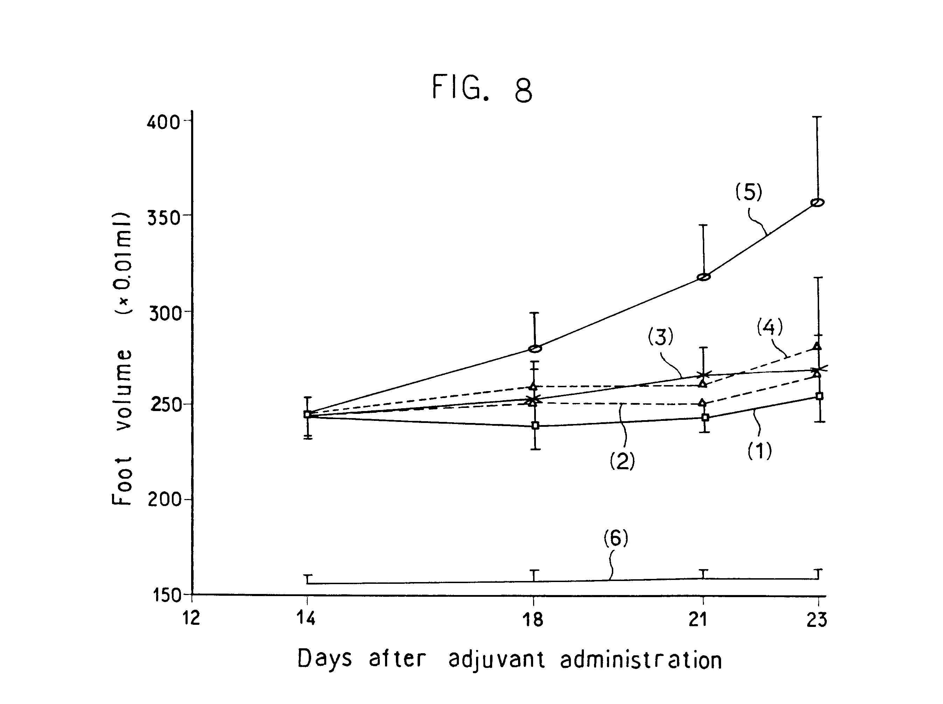

The results are shown in Fig. 8, in which the number of days after the administration of the adjuvant is plotted as abscissa vs. the foot volume (x 0.01 ml) as ordinate. In the diagram, a group to which polypeptide I was given at a dose of 100,000 GIF units/animal is represented by (1), a group with a dose of 10,000 GIF units/animal by (2), a group with a dose of 1,000 GIF units/animal by (3), a group with a dose of 100 GIF units/animal by (4), the control group with saline by (5), and a group of normal rats by (6).

Fig. 8 shows that the foot swelling became aggravated until the 23rd days in the control group (5) but that polypeptide I was found to exhibit swelling inhibiting activity on the groups (1) to (4) to which the polypeptide was given, on the 4th days (after the administration, i.e. 18th day after the administration of the adjuvant) and during the following period. Even 4 days after the administration of the final dose (i.e. on the 23rd day after the administration of the adjuvant), the polypeptide was found still effective for preventing progress of arthritis.

Pharmacological Test Example 3

Testing derivatives of the invention for effect to induce CSF production

The following test was conducted using cell strain U-373MG (ATCC HTBI7, glioblastoma, astrocytoma, human).

Cells of the above strain were suspended in Eagle's MEM medium (product of Nissui Pharmaceutical Co.) containing 10% FCS (product of GIBCO), MEM non-essential amino acids (product of Flow) and MEM sodium pyruvate (product of Flow), to a concentration of $2 \times 10^5$ cells/ml. The substance to be tested was added in varying concentrations to portions of the suspension. Each of the mixtures was incubated in a carbon dioxide incubator at 37°C for 24 hours.

The culture supernatant was collected, and the amount of CSF produced and accumulated in the supernatant was measured using mouse bone marrow cells (Lewis, I. C. et al., J. Immunol, 128, 168 (1982)-).

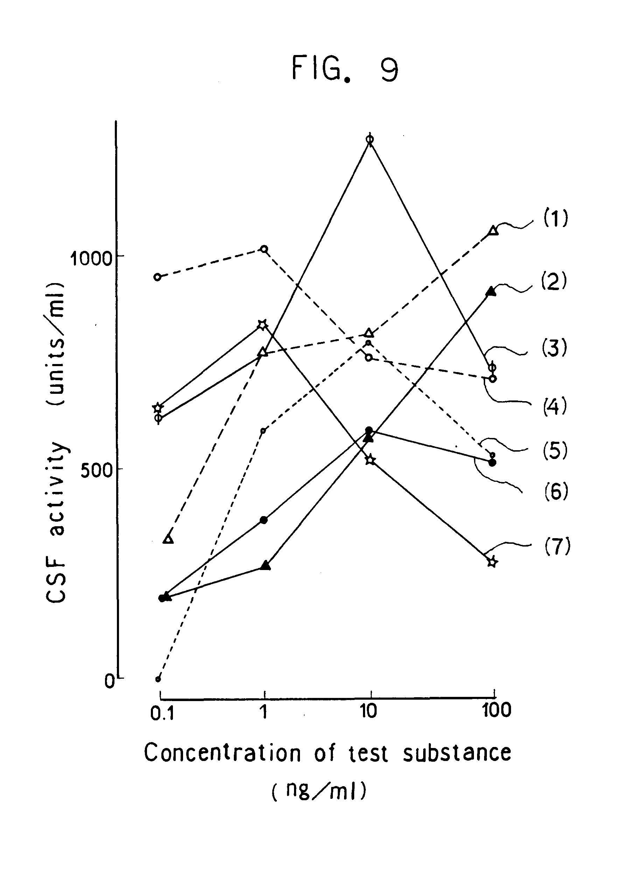

The results are given in Fig. 9, in which the concentration (ng/ml) of the test substance is plotted as abscissa vs. the CSF activity (U/ml) as ordinate. Curves (1) to (7) in the diagram represent the results achieved by the following polypeptides as test substances.

Curves (1): polypeptide VI
Curves (2): polypeptide II
Curves (3): polypeptide VIII
Curves (4): polypeptide V
Curves (5): polypeptide IV
Curves (6): polypeptide III
Curves (7): polypeptide XXX

Pharmacological Test Example 4

Testing derivative of the invention for anti-inflammatory effect

The following test was conducted according to the method of Winter et al. (Proc. Soc. Exptl. Biol. Med., 111, 544-547 (1962)).

Six- to eight-week-old male rates (Spraque Dawley strain, Nippong Charles River Co., Ltd.) were used as divided into groups of 6 to 8 rats each according to the body weight one day before the experiment. A suspension of 1% Carrageenan (product of Marine Colloid) in saline, serving as an agent for causing inflammation, was subcutaneously injected in an amount of 0.1 ml into the sole of the right rear leg of the rat to cause swelling of the foot. To evaluate the swelling, the volume of the sole was measured a predetermined period before and after the injection using a plethysmometer (product of Ugo-Vasile). The ratio of the increased volume due to the injection relative to the value before the injection was calculated as swelling %.

The substance to be tested was dissolved in Dulbeco's phosphate buffered saline, and 0.1 ml of the solution was subcutaneously injected into the back of the rat 1 hour before the injection of the inflammation causing agent. The solvent was given to a control group.

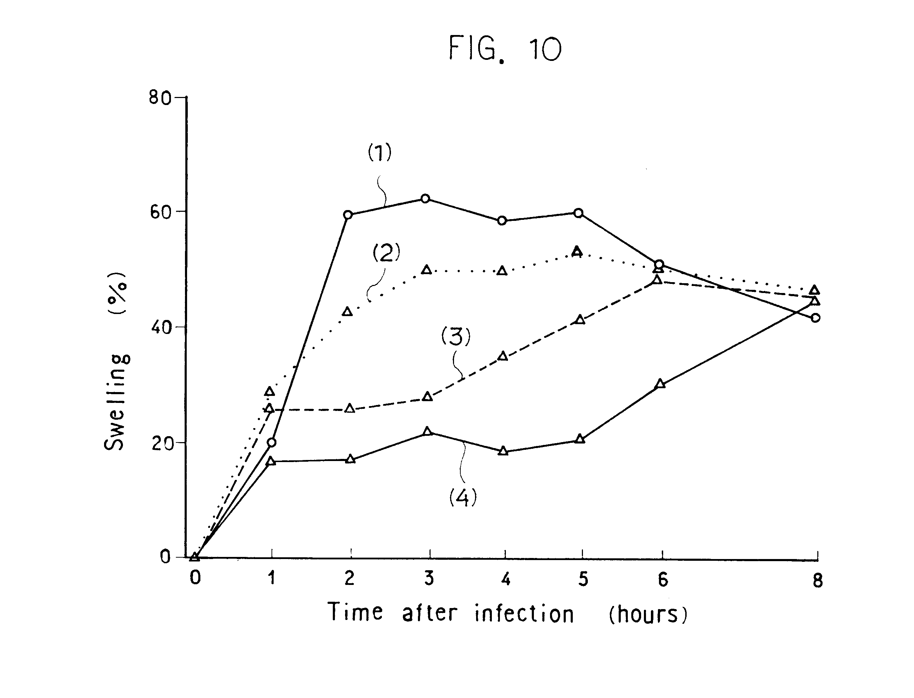

The results are shown in Fig. 10, in which the time (hours) elapsed after the injection of the inflammation causing agent is plotted as abscissa vs. the swelling % as ordinate. In the diagram, the control group is represented by curve (1), a group to which 0.1 $\mu$g of polypeptide VI was given by curve (2), a group with 1 $\mu$g of polypeptide VI by curve (3), and a group with 10 $\mu$g of polypeptide VI by curve (4).

Pharmacological Test Example 5

Testing derivative of the invention for effect to prevent radiation injury

One $\mu$g or 0.3 $\mu$g of polypeptide VI was intraperitoneally given to each of 9-week-old mice of BALB/c strain 20 hours before the mice were exposed to a lethal dosage of X-rays.

The mice were systemically exposed to X-rays at a dose of 850 roentgens by an X-ray irradiator (MBR-1505R, product of Hitachi Medico Co., Ltd.) and thereafter checked for survival daily. PBS was given to a control group.

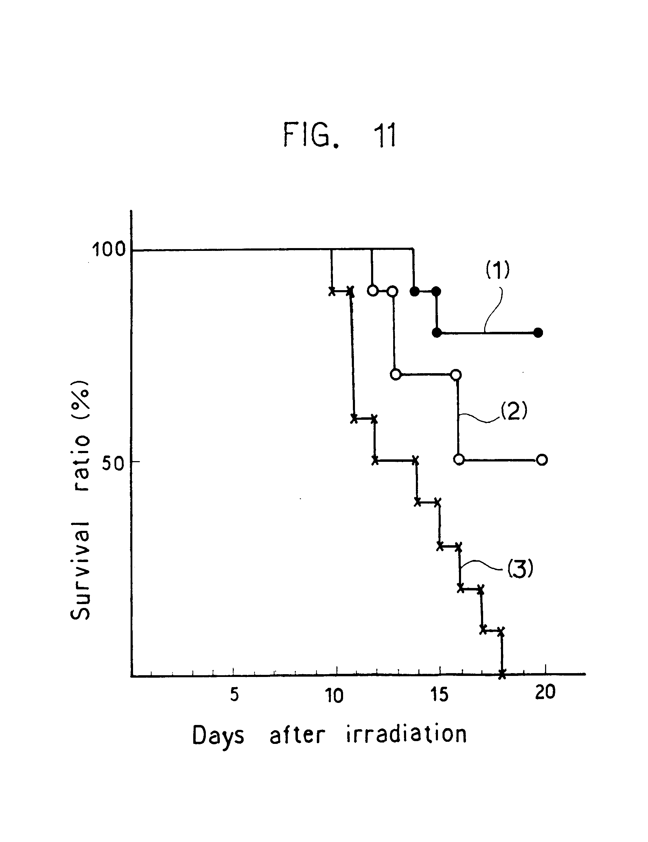

The results are shown in Fig. 11, in which the number of days after the irradiation is plotted as abscissa vs. the survival ratio (%) as ordinate. The group to which 1 $\mu$g of polypeptide VI was given is represented by curve (1), the group with 0.3 $\mu$g of polypeptide VI by curve (2), and the control group by curve (3).

Fig. 11 shows that all the mice of the control group died on the 18th day after the X-ray irradiation, whereas polypeptide VI was found effective for preventing radiation injury depending on the dosage. It was found that about 80% of the group with the dose of 1 $\mu$g were saved from death due to radiation injury and survived.

Parmacological Test Example 6

Testing derivative of the invention for effect to prevent opportunistic infection

The following test was conducted using model mouse.

On the first day, 100 mg/kg of 5-fluorouracil (5-Fu, product of Kyowa Hakko Co., Ltd.) was intravenously given to 6-week-old male mice of ICR strain (7 mice in each group). On the 2nd, 4th and 6th days, polypeptide VI of the invention was subcuteneously administered to the mice at a dose of 1 $\mu$g/mouse. On the 7th day, a specified quantity of Pseudomonas aeruginosa E-2 was intraperitoneally given to the mice for infection. On the 10th day, the number of animals survived was counted to determine the survival ratio (%).

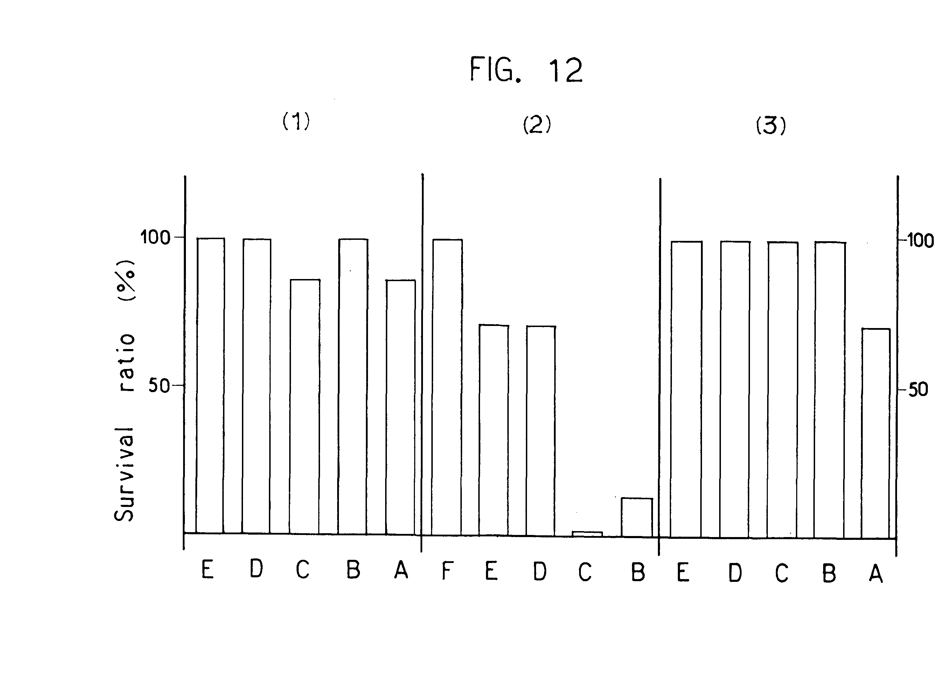

The results are given in Fig. 12, (1) to (3). Fig. 12 (1) shows the result achieved by the group thus treated. Fig. 12 (2) shows the result achieved by a control group to which polypeptide VI was not given (but 5-Fu only was given). Fig. 12 (3) shows the result achieved by another control group to which neither 5-Fu nor polypeptide VI was given.

In Fig. 12, the survival ratio (%) is plotted as ordinate vs. groups A to E which were each given the following amount of the pseudomonas.

| Group | Number of cells/mouse |
|---|---|
| A | 19,000 |
| B | 3,800 |
| C | 750 |
| D | 150 |
| E | 30 |
| F | 6 |

Preparation Example 1

To a solution of 1 x 10$^7$ units/ml, calculated as GIF activity, of polypeptide VI in saline was added human serum albumin (HSA) to a concentration of 0.5%. The mixture was filtered (0.22 $\mu$m membrane filter), placed into vials, 1 ml in each, in sterile state, and lyophilized to obtain a preparation for injection.

The preparation is used as dissolved in 1 ml of distilled water for injection.

⟨Method of preparing cytokines from animal cells⟩

(1) HSB-2C5B2 cells (J. Immunol., 131, 1682-1689 (1985)), in an amount of 2 x $10^5$ cells/well, were incubated for 24 hours in the presence of varying concentrations of polypeptide XXXVII and 0.01% PHA-P. The IL-2 activity of the supernatant collected was measured by the method of K. A. Smith et al. using IL-2 dependent mouse T cells (CTLL 2) (J. Immunol., 120, 2027 (1978)). Table 6 below shows the results.

Table 6

| Concentration of polypeptide XXXVII (mg/ml) | IL-2 activity (cpm x $10^{-3}$) mean (n = 5) |
|---|---|
| 0 | 0.2 |
| 0.0005 | 0.3 |
| 0.005 | 1.3 |
| 0.05 | 3.9 |
| 0.5 | 3.1 |
| 5 | 4.1 |
| 50 | 4.2 |
| 500 | 4.9 |

(2) The human astroglioma cell line U373MG was cultured to confluence in RPMI-1640 medium supplemental with 10% fetal calf serum and then was further incubated in fresh RPMI-1640 medium supplemented with 10% fetal calf serum and 20 ng/ml of the polypeptide XXXVII. After 18 hours, the medium was discarded, and total RNA was extracted by using the guanidinium-CsCl method, and poly $(A)^+$ RNA was selected by oligo-dT-cellulose chromatography. Ten $\mu$g of poly $(A)^+$ RNA was fractionated through 1.2% agarose gel in accordance with Northern blotting method and then was transferred to nitrocellulose filter. The blot was baked at 80°C under reduced pressure and treated in 20 mM Tris-HCl (pH 8.0) at 100°C for 5 min., following prehybridization at 42°C in 50% formamide -5 x SSC - 50 mM sodium phosphate (pH 6.5) - 4•X Denhardt's solution - 200 $\mu$g/ml denatured salmon sperm DNA. After 5 hours, it was hybridized at 42°C for 20 hours with PstI-NcoI DNA fragment of cDNA insert of GM-CSF (Science, 228, 810 (1985)) or KpnI-BamHI DNA fragment of cDNA insert of BSF-2 (Nature, 324, 73 (1986)), which fragment had been radio labelled by nick translation. The filter was washed in 2 x SSC - 0.1% SDS at room temperature for 15 min and then washed in 0.1 x SSC-0.1% SDS at 50°C for 1 hour. Autoradiography was achieved at -70°C overnight in the presence of the intensifying screen.

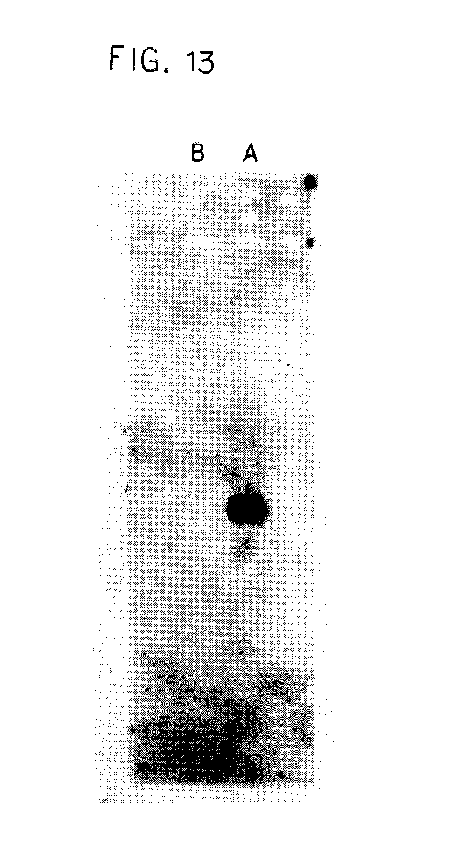

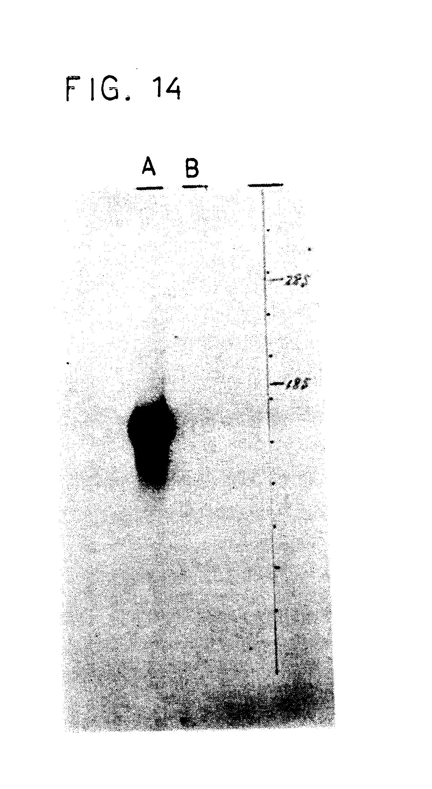

Fig. 13 shows the results achieved when the DNA fragment of GM-CSF was used. Lane A represents the result attained by the use of the present polypeptide, and lane B the result of control without using the present polypeptide. Like Fig. 13, Fig. 14 shows the results achieved when the DNA fragment of BSF-2 was used.

These results indicate that the use of the polypeptide of the invention permits animal cells to produce natural-type cytokines efficiently.

The amount of the present polypeptide to be used for the above method can be very small, usually about 10 ng/ml, whereby satisfactory results are achievable, while the use of the polypeptide facilitates purification of the cytokine derived.

(3) When cytokines are to be produced from animal cells, it is essential that the polylpeptide of the invention used for inducing the production be stable in structure under the prevailing condition and be bound to the IL-1 receptor on the surface of the cells. More specifically, it is required that the present polypeptide binds to the IL-1 receptor and transmits to the cell a singal necessary for the production of the cytokine.

Accordingly, the following test was conducted in connection with the binding of the present polypeptide to the IL-1 receptor on fibroblasts.

BALB/3T3 cells (clone A31: ATCC, CCL-163, 1 x $10^6$ cells/well) almost uniformly grown over a 6-well plate were reacted at 4°C for 2 hours with 50000 cpm/well of [125]I-labelled polypeptide I (IL-1$\beta$) and 20 ng/ml of polypeptide I preincubated at 37°C in D-MEM supplemented with 10% FCS. The liquid reaction mixture was discarded with a Pasteur pipette, the cells were gently washed with 1 ml of D-MEM supplemented with 10% FCS, and the supernatant was discarded. After repeating the washing procedure twice, the cells were solubilized with 1 ml of a mixture of 1% SDS and 0.2 N NaOH. The radioactivity (bound radioactivity) of the solubilized cell solution and the liquid used for washing the wells was measured by a gamma-counter.

The [125]I-labeled polypeptide I used was prepared and purified by the method of Bolton and Hunter (Biochem. J., 133, 529 (1973)). Specific activity: at least 250 uCi/μg protein. Table 7 below shows the results.

## Table 7

| Preincubation time (hr) | Inhibition activity (%) |
|---|---|
| 0 | 100 |
| 5 | About 40 |
| 30 | About 4 |

$$\text{Inhibition Activity (\%)} = \frac{A - B}{A - C} \times 100$$

wherein A: Bound radioactivity in absence of unlabelled polypeptide I

B: Experimental value of bound radioactivity

C: Radioactivity unspecifically absorbed on the plate

The foregoing index expresses the binding activity of the coexisting polypeptide I against IL-1 receptor.

Table 7 reveals that under the cytokine induction condition, the ability of polypeptide I, i.e. IL-1$\beta$ itself, to bind to the IL-1 receptor diminishes with time. Accordingly, the same testing procedure as above was repeated using polypeptide I, VI or XXXVII which was preincubated for 24 hours. Table 8 below shows the results.

Table 8

| Polypeptide | Inhibition activity (%) |
|---|---|
| I | About 4 |
| VI | About 44 |
| XXXVII | About 87 |

The results given above reveal that it is more preferable to use polypeptides of the invention than IL-1$\beta$ itself for preparing cytokines from animal cells.

## Claims

1. A polypeptide characterized in that the polypeptide has a modified amino acid sequence fulfilling at least one of said requirements a) to c) or at least one of said requirements a) to c) together with the requirement d) in the amino acid sequence of interleukin-1$\beta$ represented by the formula (A).

EP 0 237 967 B1

Formula (A)

```
                      5                                10
Ala - Pro - Val - Arg - Ser - Leu - Asn - Cys - Thr - Leu -

                      15                               20
Arg - Asp - Ser - Gln - Gln - Lys - Ser - Leu - Val - Met-

                      25                               30
Ser - Gly - Pro - Tyr - Glu - Leu - Lys - Ala - Leu - His-

                      35                               40
Leu - Gln - Gly - Gln - Asp - Met - Glu - Gln - Gln - Val-

                      45                               50
Val - Phe - Ser - Met - Ser - Phe - Val - Gln - Gly - Glu-

                      55                               60
Glu - Ser - Asn - Asp - Lys - Ile - Pro - Val - Ala - Leu-

                      65                               70
Gly - Leu - Lys - Glu - Lys - Asn - Leu - Tyr - Leu - Ser-

                      75                               80
Cys - Val - Leu - Lys - Asp - Asp - Lys - Pro - Thr - Leu-

                      85                               90
Gln - Leu - Glu - Ser - Val - Asp - Pro - Lys - Asn - Tyr-

                      95                               100
Pro - Lys - Lys - Lys - Met - Glu - Lys - Arg - Phe - Val-

                      105                              110
Phe - Asn - Lys - Ile - Glu - Ile - Asn - Asn - Lys - Leu-

                      115                              120
Glu - Phe - Glu - Ser - Ala - Gln - Phe - Pro - Asn - Trp-


                      125                              130
Tyr - Ile - Ser - Thr - Ser - Gln - Ala - Glu - Asn - Met-

                      135                              140
Pro - Val - Phe - Leu - Gly - Gly - Thr - Lys - Gly - Gly-

                      145                              150
Gln - Asp - Ile - Thr - Asp - Phe - Thr - Met - Gln - Phe-


Val - Ser - Ser
```

a) At least one amino acid residue selected from among Ala at the 1-position, Val at the 3-position, Arg at the 4-position, Ser at the 5-position, Cys at the 8-position, Arg at the 11-position, His at the 30-position, Cys at the 71-position, Lys at the 93-position, Lys at the 97-position, Arg at the 98-position, Phe at the 99-position, Lys at the 103-position, Trp at the 120-position, Tyr at the 121-position and Ser at the 153-position is missing or replaced by another amino acid.

45

b) The amino acid sequence of Ala at the 1-position to Thr at the 9-position or at least one amino acid residue in this sequence is missing (except that at least one amino acid residue selected from the group consisting of Ala at the 1-position, Val at the 3-position, Arg at the 4-position, Ser at the 5-position and Cys at the 8-position is missing as stated in the requirement a)).

c) The amino acid sequence of Lys at the 103 position to Ser at the 153-position or at least one amino acid residue in this sequence is missing (except that at least one amino acid residue selected from the group consisting of Lys at the 103-position, Trp at the 120-position, Tyr at the 121-position and Ser at the 153-position is missing as stated in the requirement a)).

d) Met, or the amino acid sequence of Met at the 1'-position to Asp at the 116'-position represeted by the following formula (B), or a portion of the sequence of the formula (B) toward its C terminal is attached to the N terminal of the formula (A)

Formula (B)

```
                              5'                        10'
Met - Ala - Glu - Val - Pro - Glu - Leu - Ala - Ser - Glu-

                              15'                       20'
Met - Met - Ala - Tyr - Tyr - Ser - Gly - Asn - Glu - Asp-

                              25'                       30'
Asp - Leu - Phe - Phe - Glu - Ala - Asp - Gly - Pro - Lys-

                              35'                       40'
Gln - Met - Lys - Cys - Ser - Phe - Gln - Asp - Leu - Asp-

                              45'                       50'
Leu - Cys - Pro - Leu - Asp - Gly - Gly - Ile - Gln - Leu-

                              55'                       60'
Arg - Ile - Ser - Asp - His - His - Tyr - Ser - Lys - Gly-

                              65'                       70'
Phe - Arg - Gln - Ala - Ala - Ser - Val - Val - Val - Ala-

                              75'                       80'
Met - Asp - Lys - Leu - Arg - Lys - Met - Leu - Val - Pro-

                              85'                       90'
Cys - Pro - Gln - Thr - Phe - Gln - Glu - Asn - Asp - Leu-

                              95'                       100'
Ser - Thr - Phe - Phe - Pro - Phe - Ile - Phe - Glu - Glu-


                              105'                      110'
Glu - Pro - Ile - Phe - Phe - Asp - Thr - Trp - Asp - Asn-

                              115'
Glu - Ala - Tyr - Val - His - Asp
```

provided that Cys at 71 position only is not replaced by Ser.

2. A polypeptide as defined in claim 1 which fulfills at least one of said requirements a) to c).

EP 0 237 967 B1

3. A polypeptide as defined in claim 2 wherein at least Arg at the 4-position is missing or replaced by another amino acid.

4. A polypeptide as defined in claim 3 wherein said another amino acid is Gly.

5. A polypeptide as defined in claim 3 wherein said Arg at the 4-position is missing or replaced by another amino acid.

6. A polypeptide as defined in claim 2 wherein at least Cys at the 71-position is missing or replaced by another amino acid.

7. A polypeptide as defined in claim 6 wherein said another amino acid is Ser, Ala or Val.

8. A polypeptide as defined in claim 6 wherein said Cys at the 71-position is missing or replaced by another amino acid.

9. A polypeptide as defined in claim 2 wherein said Cys at the 8-position and said Cys at the 71-position are missing or are each replaced by another amino acid.

10. A polypeptide as defined in claim 1 which is one member selected from the group consisting of polypeptides II to V, VII to IX polypeptides XI to XXX and polypeptides XXXV to XXXXVIII.

11. A polypeptide as defined in claim 10 which is polypeptide II.

12. A polypeptide as defined in claim 10 which is polypeptide XII.

13. A polypeptide as defined in claim 10 which is polypeptide XXXVII.

14. A gene coding for a polypeptide as defined in claim 2.

15. A gene coding for a polypeptide as defined in claim 11.

16. A gene coding for a polypeptide as defined in claim 12.

17. A gene coding for a polypeptide as defined in claim 13.

18. A vector containing a gene as defined in claim 14.

19. A microorganism containing a vector as defined in claim 18.

20. A medicinal composition comprising a pharmacologically effective amount of a polypeptide as defined in claim 1 and a medicinal auxiliary agent.

21. A medical composition as defined in claim 20 which is a composition for stimulating immunity.

22. A medical composition as defined in claim 20 which is a composition for curing a malignant tumor.

23. A medical composition as defined in claim 20 which is a composition for promoting production of a cytokine.

24. A medical composition as defined in claim 20 which is a composition for curing inflammation.

25. A medical composition as defined in claim 20 which is a composition for preventing or curing radiation injury.

26. A medical composition as defined in claim 20 which is a composition for preventing or curing opportunistic infection.

47

**27.** A medical composition as defined in claim 20, which is a composition for promoting production of CSF.

**Patentansprüche**

**1.** Polypeptid, dadurch gekennzeichnet, daß das Polypeptid eine modifizierte Aminosäuresequenz aufweist, die mindestens eines der Erfordernisse a) bis c) oder mindestens eines der Erfordernisse a) bis c) zusammen mit dem Erfordernis d) in der Aminosäuresequenz von Interleukin-1$\beta$ gemäß der Formel (A) erfüllt,

**Formel (A)**

```
                         5                          10
Ala - Pro - Val - Arg - Ser - Leu - Asn - Cys - Thr - Leu -

                        15                          20
Arg - Asp - Ser - Gln - Gln - Lys - Ser - Leu - Val - Met-

                        25                          30
Ser - Gly - Pro - Tyr - Glu - Leu - Lys - Ala - Leu - His-

                        35                          40
Leu - Gln - Gly - Gln - Asp - Met - Glu - Gln - Gln - Val-

                        45                          50
Val - Phe - Ser - Met - Ser - Phe - Val - Gln - Gly - Glu-

                        55                          60
Glu - Ser - Asn - Asp - Lys - Ile - Pro - Val - Ala - Leu-

                        65                          70
Gly - Leu - Lys - Glu - Lys - Asn - Leu - Tyr - Leu - Ser-

                        75                          80
Cys - Val - Leu - Lys - Asp - Asp - Lys - Pro - Thr - Leu-

                        85                          90
Gln - Leu - Glu - Ser - Val - Asp - Pro - Lys - Asn - Tyr-

                        95                         100
Pro - Lys - Lys - Lys - Met - Glu - Lys - Arg - Phe - Val-

                       105                         110
Phe - Asn - Lys - Ile - Glu - Ile - Asn - Asn - Lys - Leu-

                       115                         120
Glu - Phe - Glu - Ser - Ala - Gln - Phe - Pro - Asn - Trp-
```

48

```
                    125                                    130
Tyr - Ile - Ser - Thr - Ser - Gln - Ala - Glu - Asn - Met-

                    135                                    140
Pro - Val - Phe - Leu - Gly - Gly - Thr - Lys - Gly - Gly-

                    145                                    150
Gln - Asp - Ile - Thr - Asp - Phe - Thr - Met - Gln - Phe-


Val - Ser - Ser
```

nämlich

a) mindestens ein Aminosäurerest, ausgewählt aus Ala in Position 1, Val in Position 3, Arg in Position 4, Ser in Position 5, Cys in Position 8, Arg in Position 11, His in Position 30, Cys in Position 71, Lys in Position 93, Lys in Position 97, Arg in Position 98, Phe in Position 99, Lys in Position 103, Trp in Position 120, Tyr in Position 121 und Ser in Position 153, fehlt oder ist durch eine andere Aminosäure ersetzt,

b) die Aminosäuresequenz von Ala in Position 1 bis Thr in Position 9 oder mindestens ein Aminosäurerest in dieser Sequenz fehlt (außer daß mindestens ein Aminosäurerest, ausgewählt aus Ala in Position 1, Val in Position 3, Arg in Position 4, Ser in Position 5 und Cys in Position 8, fehlt, wie in Erfordernis a) dargelegt),

c) die Aminosäuresequenz von Lys in Position 103 bis Ser in Position 153 oder mindestens ein Aminosäurerest in dieser Sequenz fehlt (außer daß mindestens ein Aminosäurerest, ausgewählt aus Lys in Position 103, Trp in Position 120, Tyr in Position 121 und Ser in Position 153, fehlt, wie in Erfordernis a) dargelegt),

d) Met oder die Aminosäuresequenz von Met in der Position 1' bis Asp in der Position 116' gemäß der folgenden Formel (B) oder ein Teil der Sequenz der Formel (B) in Richtung ihres C-terminalen Endes ist an das N-terminale Ende der Formel (A) geknüpft

Formel (B)

```
                                  5'                                      10'
   Met - Ala - Glu - Val - Pro - Glu - Leu - Ala - Ser - Glu-

                                 15'                                      20'
   Met - Met - Ala - Tyr - Tyr - Ser - Gly - Asn - Glu - Asp-

                                 25'                                      30'
   Asp - Leu - Phe - Phe - Glu - Ala - Asp - Gly - Pro - Lys-

                                 35'                                      40'
   Gln - Met - Lys - Cys - Ser - Phe - Gln - Asp - Leu - Asp-

                                 45'                                      50'
   Leu - Cys - Pro - Leu - Asp - Gly - Gly - Ile - Gln - Leu-

                                 55'                                      60'
   Arg - Ile - Ser - Asp - His - His - Tyr - Ser - Lys - Gly-

                                 65'                                      70'
   Phe - Arg - Gln - Ala - Ala - Ser - Val - Val - Val - Ala-

                                 75'                                      80'
   Met - Asp - Lys - Leu - Arg - Lys - Met - Leu - Val - Pro-

                                 85'                                      90'
   Cys - Pro - Gln - Thr - Phe - Gln - Glu - Asn - Asp - Leu-

                                 95'                                     100'
   Ser - Thr - Phe - Phe - Pro - Phe - Ile - Phe - Glu - Glu-

                                105'                                     110'
   Glu - Pro - Ile - Phe - Phe - Asp - Thr - Trp - Asp - Asn-

                                115'
   Glu - Ala - Tyr - Val - His - Asp
```

mit der Maßgabe, daß nur Cys in Position 71 nicht durch Ser ersetzt ist.

**2.** Polypeptid nach Anspruch 1, das mindestens eines der Erfordernisse a) bis c) erfüllt.

**3.** Polypeptid nach Anspruch 2, wobei mindestens Arginin in Position 4 fehlt oder durch eine andere Aminosäure ersetzt ist.

**4.** Polypeptid nach Anspruch 3, wobei die andere Aminosäure Gly ist.

**5.** Polypeptid nach Anspruch 3, wobei das Arg in Position 4 fehlt oder durch eine andere Aminosäure ersetzt ist.

**6.** Polypeptid nach Anspruch 2, wobei mindestens Cys in Position 71 fehlt oder durch eine andere Aminosäure ersetzt ist.

**7.** Polypeptid nach Anspruch 6, wobei die andere Aminosäure Ser, Ala oder Val ist.

50

**8.** Polypeptid nach Anspruch 6, wobei das Cys in Position 71 fehlt oder durch eine andere Aminosäure ersetzt ist.

**9.** Polypeptid nach Anspruch 2, wobei das Cys in Position 8 und das Cys in Position 71 fehlen oder jeweils durch eine andere Aminosäure ersetzt sind.

**10.** Polypeptid nach Anspruch 1, ausgewählt aus den Polypeptiden II bis V, VII bis IX, den Polypeptiden XI bis XXX und den Polypeptiden XXXV bis XXXXVIII.

**11.** Polypeptid nach Anspruch 10, das das Polypeptid II ist.

**12.** Polypeptid nach Anspruch 10, das das Polypeptid XII ist.

**13.** Polypeptid nach Anspruch 10, das das Polypeptid XXXVII ist.

**14.** Gen, das ein Polypeptid nach Anspruch 2 codiert.

**15.** Gen, das ein Polypeptid nach Anspruch 11 codiert.

**16.** Gen, das ein Polypeptid nach Anspruch 12 codiert.

**17.** Gen, das ein Polypeptid nach Anspruch 13 codiert.

**18.** Vektor, der ein Gen nach Anspruch 14 enthält.

**19.** Mikroorganismus, der einen Vektor nach Anspruch 18 enthält.

**20.** Medizinische Zusammensetzung, umfassend eine pharmakologisch wirksame Menge eines Polypeptids nach Anspruch 1 und ein medizinisches Hilfsmittel.

**21.** Medizinische Zusammensetzung nach Anspruch 20, die eine Zusammensetzung zur Stimulierung der Immunität ist.

**22.** Medizinische Zusammensetzung nach Anspruch 20, die eine Zusammensetzung zur Heilung eines malignen Tumors ist.

**23.** Medizinische Zusammensetzung nach Anspruch 20, die eine Zusammensetzung zur Förderung der Produktion eines Cytokins ist.

**24.** Medizinische Zusammensetzung nach Anspruch 20, die eine Zusammensetzung zur Heilung einer Entzündung ist.

**25.** Medizinische Zusammensetzung nach Anspruch 20, die eine Zusammensetzung zur Vorbeugung oder Heilung einer durch Strahlung verursachten Verletzung ist.

**26.** Medizinische Zusammensetzung nach Anspruch 20, die eine Zusammensetzung zur Vorbeugung oder Heilung einer opportunistischen Infektion ist.

**27.** Medizinische Zusammensetzung nach Anspruch 20, die eine Zusammensetzung zur Förderung der Produktion von CSF ist.

**Revendications**

**1.** Polypeptide caractérisé en ce que le polypeptide a une séquence d'acides aminés modifiée satisfaisant à l'une au moins de ces conditions (a) à (c) ou à l'une au moins de ces conditions (a) à (c) avec la condition (d) dans la séquence d'acides aminés de l'interleukine-1$\beta$ représentée par la formule (A),

## Formule (A)

```
                                    5                              10
Ala - Pro - Val - Arg - Ser - Leu - Asn - Cys - Thr - Leu -

                                    15                             20
Arg - Asp - Ser - Gln - Gln - Lys - Ser - Leu - Val - Met-

                                    25                             30
Ser - Gly - Pro - Tyr - Glu - Leu - Lys - Ala - Leu - His-

                                    35                             40
Leu - Gln - Gly - Gln - Asp - Met - Glu - Gln - Gln - Val-

                                    45                             50
Val - Phe - Ser - Met - Ser - Phe - Val - Gln - Gly - Glu-

                                    55                             60
Glu - Ser - Asn - Asp - Lys - Ile - Pro - Val - Ala - Leu-

                                    65                             70
Gly - Leu - Lys - Glu - Lys - Asn - Leu - Tyr - Leu - Ser-

                                    75                             80
Cys - Val - Leu - Lys - Asp - Asp - Lys - Pro - Thr - Leu-

                                    85                             90
Gln - Leu - Glu - Ser - Val - Asp - Pro - Lys - Asn - Tyr-

                                    95                             100
Pro - Lys - Lys - Lys - Met - Glu - Lys - Arg - Phe - Val-

                                    105                            110
Phe - Asn - Lys - Ile - Glu - Ile - Asn - Asn - Lys - Leu-

                                    115                            120
Glu - Phe - Glu - Ser - Ala - Gln - Phe - Pro - Asn - Trp-

                                    125                            130
    Tyr - Ile - Ser - Thr - Ser - Gln - Ala - Glu - Asn - Met-

                                    135                            140
    Pro - Val - Phe - Leu - Gly - Gly - Thr - Lys - Gly - Gly-

                                    145                            150
    Gln - Asp - Ile - Thr - Asp - Phe - Thr - Met - Gln - Phe-

    Val - Ser - Ser
```

à savoir

(a) au moins un résidu d'acide aminé choisi parmi Ala à la position 1, Val à la position 3, Arg à la position 4, Ser à la position 5, Cys à la position 8, Arg à la position 11, His à la position 30, Cys à la position 71, Lys à la position 93, Lys à la position 97, Arg à la position 98, Phe à la position 99, Lys à la position 103, Trp à la position 120, Tyr à la position 121 et Ser à la position 153, est absent ou remplacé par un autre acide aminé.

(b) la séquence d'acides aminés allant de Ala à la position 1 jusqu'à Thr à la position 9 ou bien au moins un résidu d'acide aminé dans cette séquence est absent [sauf qu'au moins un résidu d'acide aminé choisis dans le coupe consistant en Ala à la position 1, Val à la position 3, Arg à la position 4, Ser à la position 5 et Cys à la position 8, est absent comme cela est indiqué dans la condition (a)],

(c) la séquence d'acides aminés allant de Lys à la position 103 jusqu'à Ser à la position 153 ou bien au moins un résidu d'acide aminé dans cette séquence est absent [sauf qu'au moins un résidu d'acide aminé choisi dans le coupe consistant en Lys à la position 103, Trp à la position 120, Tyr à la position 121 et Ser à la position 153, est absent comme cela est indiqué dans la condition (a)],

(d) Met, ou la séquence d'acides aminés de Met à la position 1' jusqu'à Asp à la position 116' représentée par la formule suivante (B), ou une portion de la séquence de formule (B) vers son C terminal, est attaché au N terminal de la formule (A),

## Formule (B)

```
                              5'                          10'
Met - Ala - Glu - Val - Pro - Glu - Leu - Ala - Ser - Glu-

                              15'                         20'
Met - Met - Ala - Tyr - Tyr - Ser - Gly - Asn - Glu - Asp-

                              25'                         30'
Asp - Leu - Phe - Phe - Glu - Ala - Asp - Gly - Pro - Lys-

                              35'                         40'
Gln - Met - Lys - Cys - Ser - Phe - Gln - Asp - Leu - Asp-

                              45'                         50'
Leu - Cys - Pro - Leu - Asp - Gly - Gly - Ile - Gln - Leu-

                              55'                         60'
Arg - Ile - Ser - Asp - His - His - Tyr - Ser - Lys - Gly-

                              65'                         70'
Phe - Arg - Gln - Ala - Ala - Ser - Val - Val - Val - Ala-

                              75'                         80'
Met - Asp - Lys - Leu - Arg - Lys - Met - Leu - Val - Pro-

                              85'                         90'
Cys - Pro - Gln - Thr - Phe - Gln - Glu - Asn - Asp - Leu-

                              95'                         100'
Ser - Thr - Phe - Phe - Pro - Phe - Ile - Phe - Glu - Glu-

                              105'                        110'
Glu - Pro - Ile - Phe - Phe - Asp - Thr - Trp - Asp - Asn-

                              115'
Glu - Ala - Tyr - Val - His - Asp
```

à condition que Cys à la position 71 uniquement ne soit pas remplacé par Ser.

**2.** Polypeptide suivant la revendication 1, qui satisfait au moins à l'une de ces conditions (a) à (c).

**3.** Polypeptide suivant la revendication 2, dans lequel au moins Arg à la position 4 est absent ou remplacé par un autre acide aminé.

**4.** Polpeptide suivant la revendication 3, dans lequel cet autre acide aminé est Gly.

**5.** Polypeptide suivant la revendication 3, dans lequel cet Arg à la position 4 est absent ou remplacé par un autre acide aminé.

**6.** Polypeptide suivant la revendication 2, dans lequel au moins Cys à la position 71 est absent ou remplacé par un autre acide aminé.

**7.** Polypeptide suivant la revendication 6, dans lequel cet autre acide aminé est Ser, Ala ou Val.

**8.** Polypeptide suivant la revendication 6, dans lequel ce Cys à la position 71 est absent ou remplacé par un autre acide aminé.

**9.** Polypeptide suivant la revendication 2, dans lequel ce Cys à la position 8 et ce Cys à la position 71 sont absents ou remplacés chacun par un autre acide aminé.

**10.** Polypeptide suivant la revendication 1, qui est un membre choisi dans le coupe consistant en polypeptides II à V, VII à IX, polpeptides XI à XXX et polypeptides XXXV à XXXXVIII.

**11.** Polypeptide suivant la revendication 10, qui est le polypeptide II.

**12.** Polypeptide suivant la revendication 10, qui est le polpeptide XII.

**13.** Polypeptide suivant la revendication 10, qui est le polypeptide XXXVII.

**14.** Gène codant pour un polypeptide suivant la revendication 2.

**15.** Gène codant pour un polypeptide suivant la revendication 11.

**16.** Gène codant pour un polypeptide suivant la revendication 12.

**17.** Gène codant pour un polypeptide suivant la revendication 13.

**18.** Vecteur contenant un gène suivant la revendication 14.

**19.** Microorganisme contenant un vecteur suivant la revendication 18.

**20.** Composition médicamenteuse comprenant une quantité efficace du point de vue pharmacologique d'un polypeptide suivant la revendication 1 et un agent adjuvant médicamenteux.

**21.** Composition médicamenteuse suivant la revendication 20, qui est une composition pour stimuler l'immunité.

**22.** Composition médicamenteuse suivant la revendication 20, qui est une composition pour guérir une tumeur maligne.

**23.** Composition médicamenteuse suivant la revendication 20, qui est une composition pour activer la production d'une cytokine.

**24.** Composition médicamenteuse suivant la revendication 20, qui est une composition pour guérir une inflammation.

**25.** Composition médicamenteuse suivant la revendication 20, qui est une composition pour prévenir ou guérir une lésion par irradiation.

**26.** Composition médicamenteuse suivant la revendication 20, qui est une composition pour prévenir ou guérir une infection opportuniste.

**27.** Composition médicamenteuse suivant la revendication 20, qui est une composition pour activer la production de CSF.

FIG. 1

# FIG. 2

Pst I
ClaI HindIII
PstI
pGIFα
MspI
AccI
PvuI

ClaI
Ap
trp
pTMI
Tc
BamHI

AccI
AccI, cℓaI
1.2kb Fragment

ClaI, BamHI
about 4.4kb

DNA PolymeraseI(klenow)
BamHI Linker
BamHI
MspI

MspI-BamHI Fragment (about 540
Synthetic Oligonucleotide bp)

CℓaI, BamHI

T4 DNA Ligase

SD
Met

trp — AAGGGTATCGATAATGGCTCCTGTACGTTCTCTGAACTGCACTCTCCGG
TTCCCATAGCTATTACCGAGGACATGCAAGAGACTTGACGTGAGAGGCC
CℓaI                                                    MspI
ptrpGIF-α

EP 0 237 967 B1

FIG. 3-a

FIG. 3-b

EP 0 237 967 B1

FIG. 3-c

FIG. 3-d

# FIG. 4

pcD-GIF-16

PstI NcoI    NcoI    Hind III    Pvu II         Acc I

200    400    600    800    1000    1200    1400

pcD-GIF-207

PstI    Hind III EcoR I         Hinc II NdeI

200    400    600    800    1000    1200    1400    1500    1800

## FIG. 5

EP 0 237 967 B1

# FIG. 6

Concentration of Polypeptide I

(GIF units/ml)

# FIG. 7

FIG. 8

EP 0 237 967 B1

FIG. 9

FIG. 10

EP 0 237 967 B1

FIG. 11

# FIG. 12

EP 0 237 967 B1

FIG. 13

FIG. 14